# EUROPEAN PATENT SPECIFICATION

(11) **EP 4 691 418 B1**
(45) Date of publication and mention of the grant of the patent: **07.10.2026**
(21) Application number: 24779860.6
(22) Date of filing: 21.03.2024
(51) Int. Cl.: A61C 19/04, A61B 1/00, A61B 1/24, A61C 17/22, A61C 17/02

(54) **ORAL CARE MODE DETERMINATION DEVICE, ORAL CARE MODE DETERMINATION METHOD, AND PROGRAM**
MUNDPFLEGEMODUSBESTIMMUNGSVORRICHTUNG, MUNDPFLEGEMODUSBESTIMMUNGSVERFAHREN UND PROGRAMM
DISPOSITIF DE DÉTERMINATION DE MODE DE SOINS BUCCAUX, PROCÉDÉ DE DÉTERMINATION DE MODE DE SOINS BUCCAUX ET PROGRAMME

(30) Priority: 30.03.2023 JP 2023055154
(43) Date of publication of application: 11.02.2026
(73) Proprietor: Panasonic Intellectual Property Management Co., Ltd., Kadoma-shi, Osaka 571-0057 (JP)
(72) Inventor: OHTSUKA, Yoshio, Kadoma-shi, Osaka 571-0057 (JP); HAMASAKI, Takeshi, Kadoma-shi, Osaka 571-0057 (JP)
(74) Representative: Novagraaf International SA
(86) International application number: PCT/JP2024/011078
(87) International publication number: WO 2024/203740

(56) References cited:
- WO-A1-2014/097022
- WO-A1-2019/150515
- WO-A1-2019/150515
- WO-A2-2016/201063
- CN-A- 101 773 415
- JP-A- 2005 017 148
- JP-A- 2006 122 149
- JP-A- 2020 103 786
- JP-A- H0 833 657
- US-A1- 2022 189 611
- US-A1- 2023 010 922
- LANG NIKLAUS P., SCHÄTZLE MARC A., LÖE HARALD: "Gingivitis as a risk factor in periodontal disease", JOURNAL OF CLINICAL PERIODONTOLOGY, BLACKWELL MUNKSGAARD, COPENHAGEN, DK, vol. 36, no. s10, 1 July 2009 (2009-07-01), DK , pages 3 - 8, XP093217236, ISSN: 0303-6979, DOI: 10.1111/j.1600-051X.2009.01415.x

## Description

### [Technical Field]

The present disclosure relates to an oral care mode determination device, an oral care mode determination method, and a program.

### [Background Art]

As an oral cleaning device using a liquid such as a cleaning liquid (in other words, an oral care device for use in caring for an oral cavity), Patent Literature (PTL) 1 discloses a cleaning device that supplies a liquid containing fine bubbles to an oral cavity and integrates the step of brushing teeth and the step of rinsing, thereby cleaning the oral cavity and teeth surfaces at the same time.

### [Citation List]

### [Patent Literature]

[PTL 1] Japanese Unexamined Patent Application Publication No. 2017-118940.

WO2019150515 A1 discloses an oral care system which uses a toothbrush with an integrated imaging device to capture intraoral images, process pixel data to detect and quantify plaque and gums, and overlay the results on tooth images, enabling users to accurately identify plaque location and amount while monitoring gum health during brushing.

### [Summary of Invention]

### [Technical Problem]

Incidentally, control of the cleaning performance (in other words, care performance in caring for an oral cavity) of an oral cleaning device is important. The present disclosure provides, for example, an oral care mode determination device capable of determining a care mode, for achieving proper cleaning performance, in which an oral cleaning device is to be controlled.

### [Solution to Problem]

The invention is as defined in the appended claims.

An oral care mode determination device according to one aspect of the present disclosure includes: a dental plaque adhesion amount detector that detects, from an image of an oral cavity, a dental plaque amount that is the adhesion amount of dental plaque inside the oral cavity; a poor-condition detector that detects, from the image, a poor condition level that is the level of a poor condition of a gingiva inside the oral cavity; a care mode determiner that determines, according to the dental plaque amount detected and the poor condition level detected, a care mode that defines driving control of an oral care device for use in caring for the oral cavity; and a result outputter that outputs the determination result of the care mode to the oral care device.

An oral care mode determination method according to another aspect of the present disclosure is an oral care mode determination method performed by a computer. The oral care mode determination method includes: detecting, from an image of an oral cavity, a dental plaque amount that is the adhesion amount of dental plaque inside the oral cavity; detecting, from the image, a poor condition level that is the level of a poor condition of a gingiva inside the oral cavity; determining, according to the dental plaque amount detected and the poor condition level detected, a care mode that defines driving control of an oral care device for use in caring for the oral cavity; and outputting the determination result of the care mode to the oral care device.

A program according to still another aspect of the present disclosure is a program for causing the computer to execute the above oral care mode determination method.

### [Advantageous Effects of Invention]

By using, for example, an oral care mode determination device according to the present disclosure, it is possible to determine a care mode, for achieving proper cleaning performance, in which an oral cleaning device is to be controlled.

### [Brief Description of Drawings]

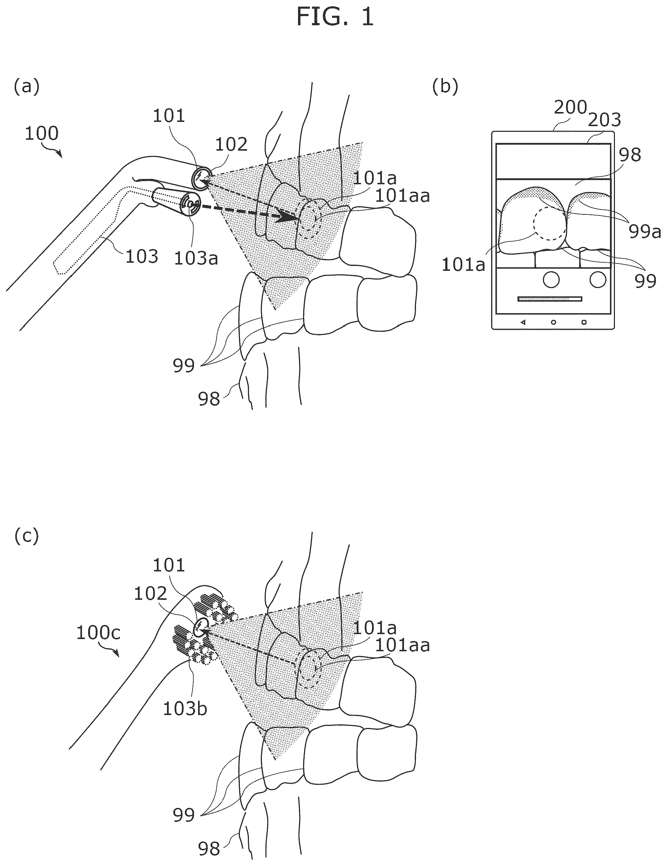

[FIG. 1] FIG. 1 illustrates a usage example of, for example, an oral cleaning device according to an embodiment.
[FIG. 2]

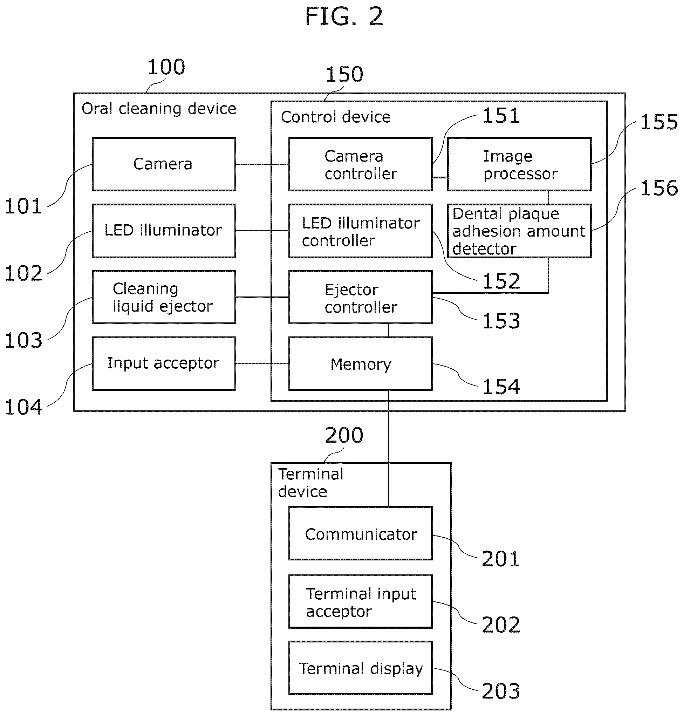

   FIG. 2 is a block diagram illustrating a functional configuration of, for example, the oral cleaning device according to the embodiment.
[FIG. 3]

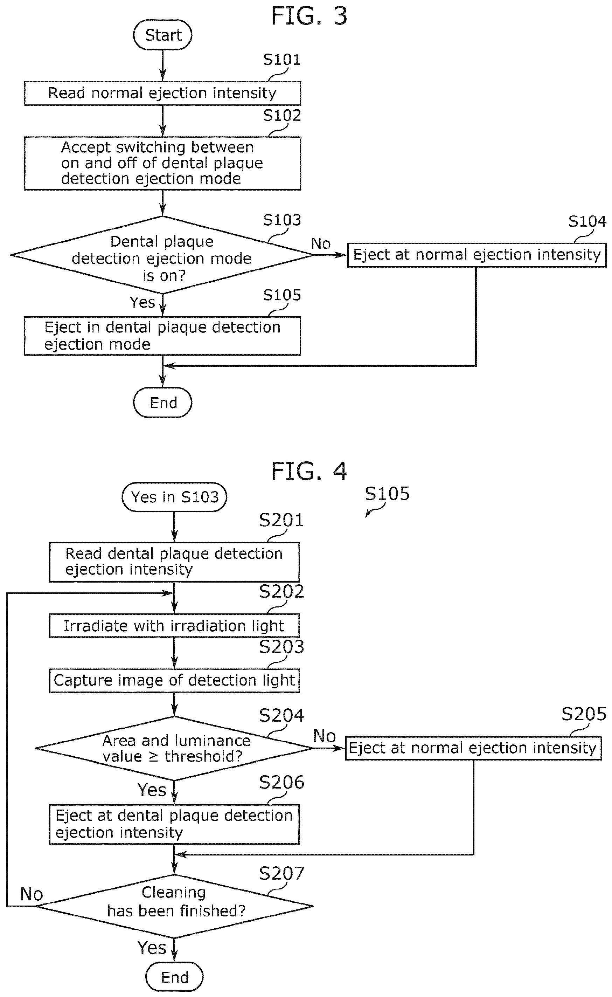

   FIG. 3 is a flowchart illustrating an operation example of the oral cleaning device according to the embodiment.
[FIG. 4]
   FIG. 4 is a flowchart illustrating an example of an operation of the oral cleaning device according to the embodiment performed in a dental plaque detection ejection mode.
[FIG. 5]

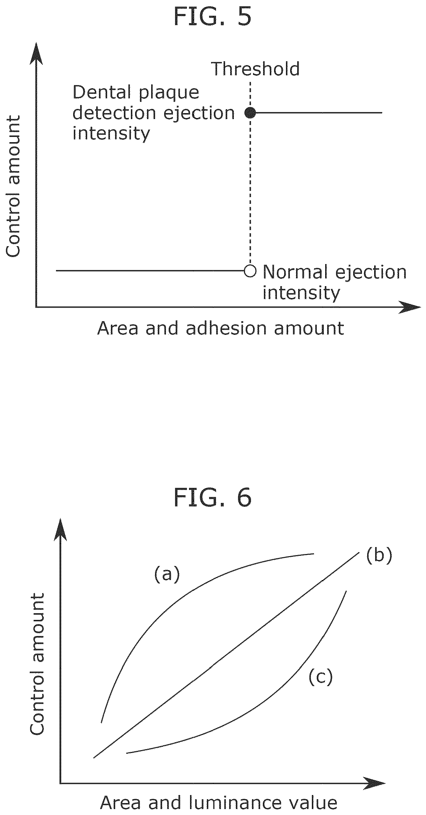

   FIG. 5 is a graph illustrating a relationship between detection results and control amounts in the oral cleaning device according to the embodiment.
[FIG. 6]
   FIG. 6 is another graph illustrating a relationship between detection results and control amounts in the oral cleaning device according to the embodiment.
[FIG. 7A]

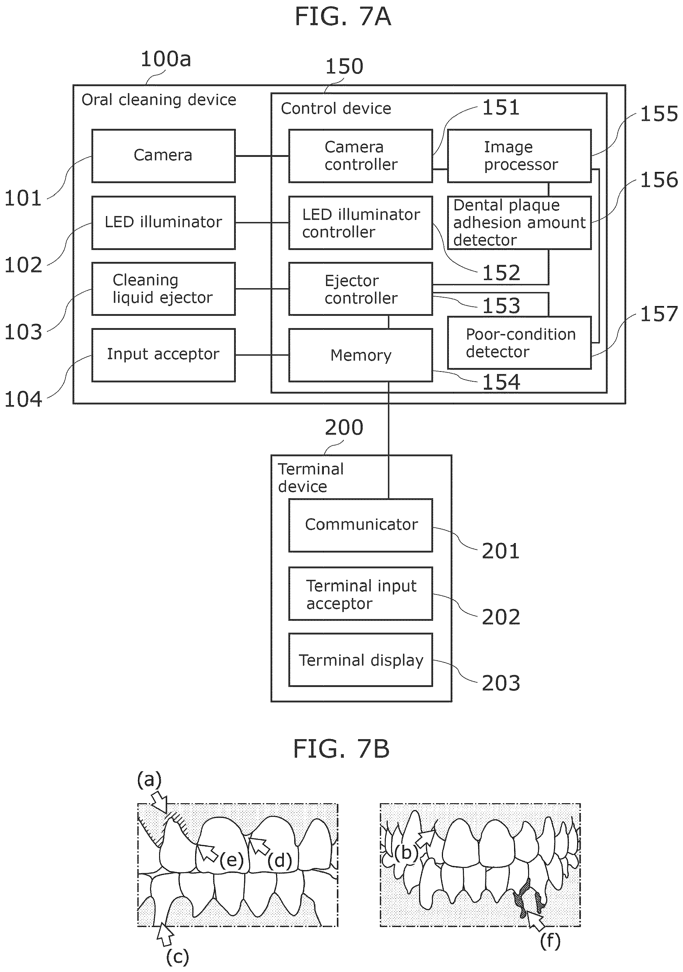

   FIG. 7A is a block diagram illustrating a functional configuration of, for example, an oral cleaning device according to Variation 1 of the embodiment.
[FIG. 7B] FIG. 7B illustrates an example of data for machine learning in the oral cleaning device according to Variation 1 of the embodiment.
[FIG. 8]

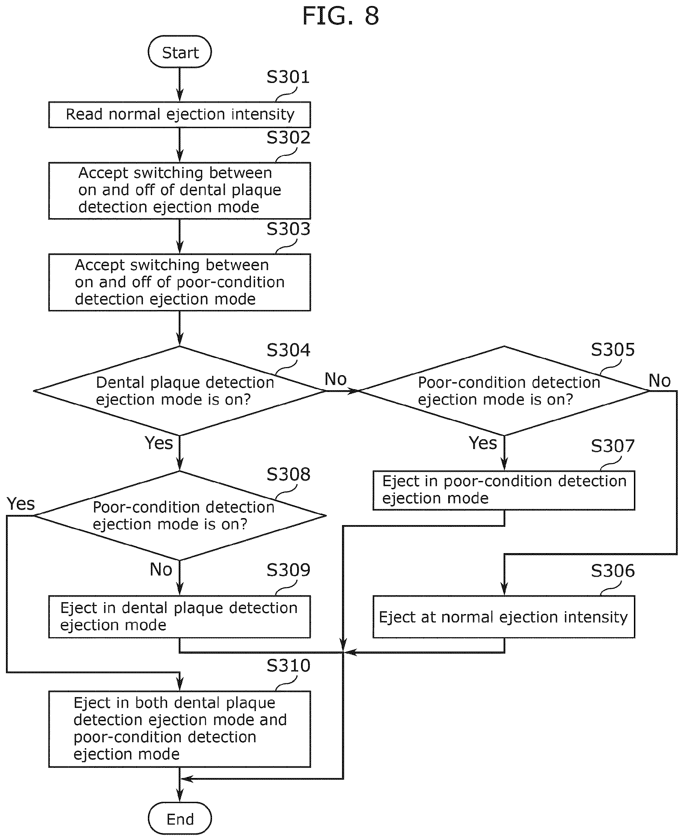

   FIG. 8 is a flowchart illustrating an operation example of the oral cleaning device according to Variation 1 of the embodiment.
[FIG. 9]

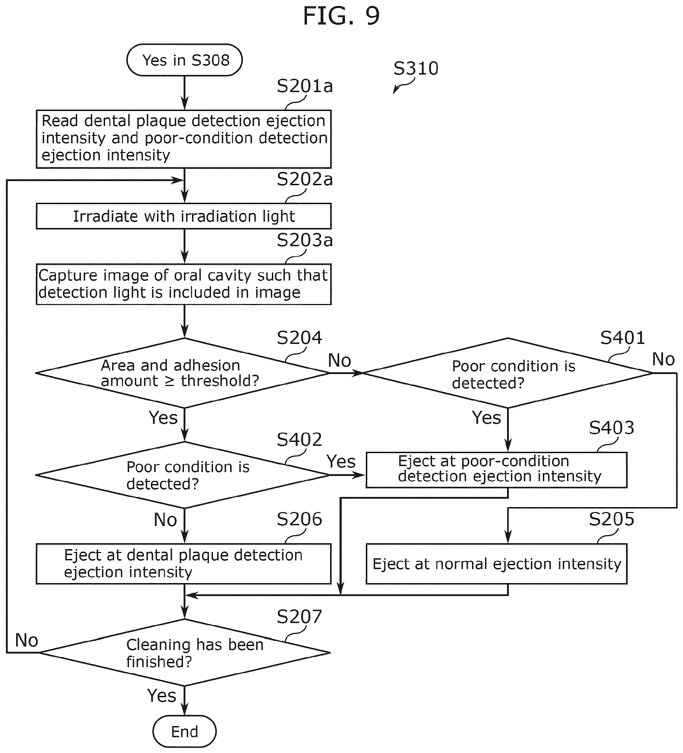

   FIG. 9 is a flowchart illustrating an example of an operation of the oral cleaning device according to Variation 1 of the embodiment performed in both the dental plaque detection ejection mode and a poor-condition detection ejection mode.
[FIG. 10]

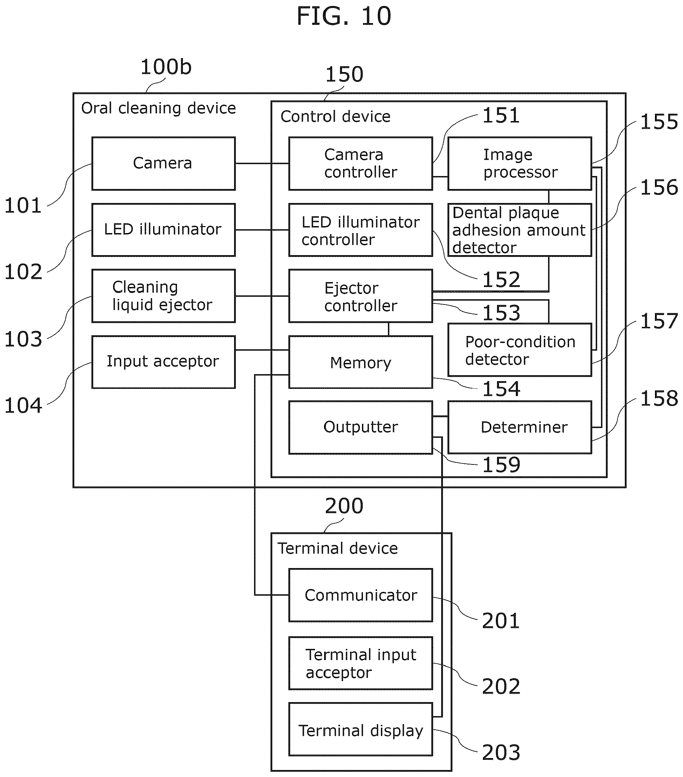

   FIG. 10 is a block diagram illustrating a functional configuration of, for example, an oral cleaning device according to Variation 2 of the embodiment.
[FIG. 11]

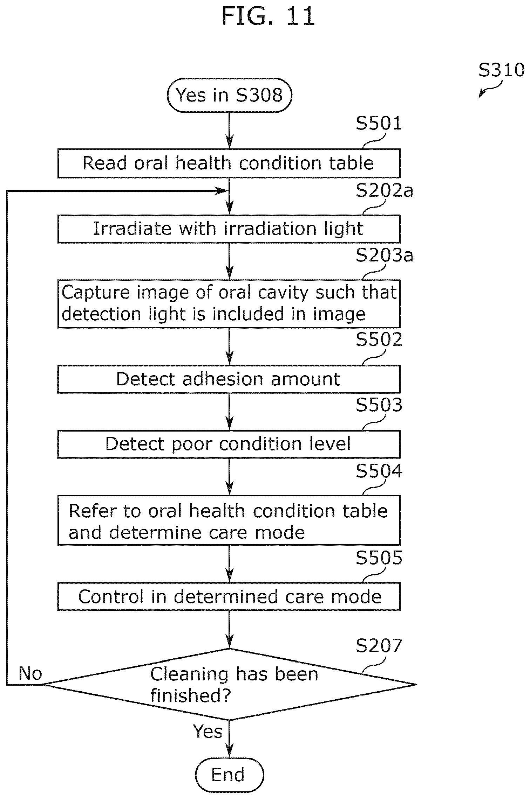

   FIG. 11 is a flowchart illustrating an example of an operation of an oral cleaning device according to Variation 3 of the embodiment performed in both the dental plaque detection ejection mode and the poor-condition detection ejection mode.
[FIG. 12]

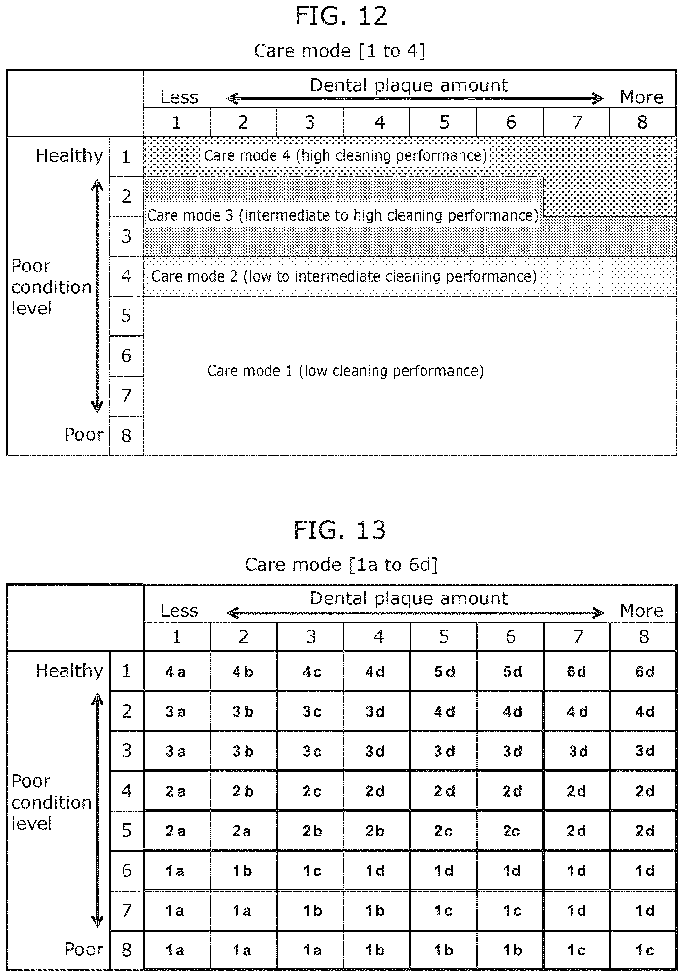

   FIG. 12 illustrates an example of an oral health condition table according to a variation of the embodiment.
[FIG. 13]
   FIG. 13 illustrates an example of an oral health condition table according to another example of the variation of the embodiment.
[FIG. 14]

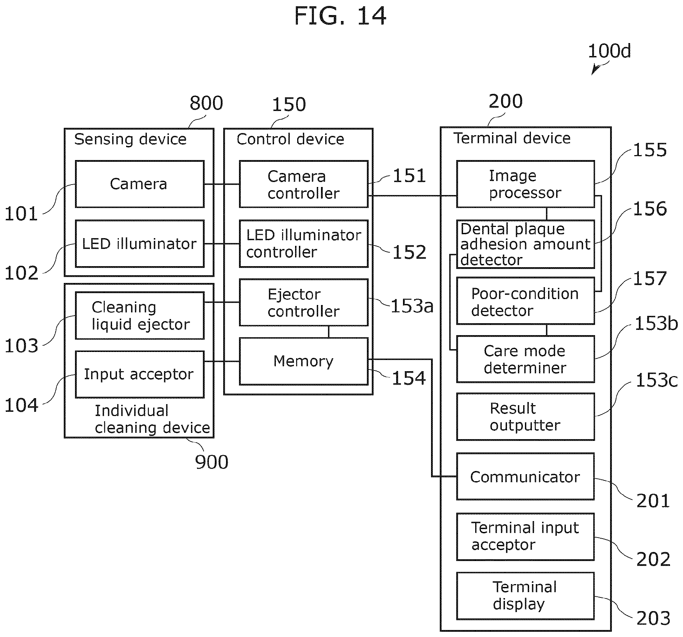

   FIG. 14 is a block diagram illustrating a functional configuration of an oral cleaning system according to Variation 4 of the embodiment.
[FIG. 15]

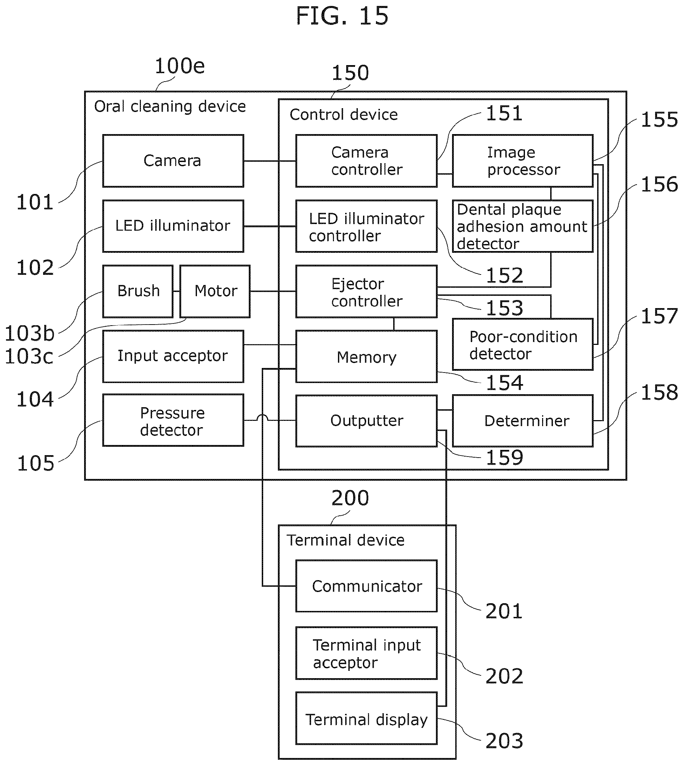

   FIG. 15 is a block diagram illustrating a functional configuration of an oral cleaning system according to Variation 5 of the embodiment.

### [Description of Embodiment]

An oral care mode determination device according to the first aspect of the present disclosure includes: a dental plaque adhesion amount detector that detects, from an image of an oral cavity, a dental plaque amount that is the adhesion amount of dental plaque inside the oral cavity; a poor-condition detector that detects, from the image, a poor condition level that is the level of a poor condition of a gingiva inside the oral cavity; a care mode determiner that determines, according to the dental plaque amount detected and the poor condition level detected, a care mode that defines driving control of an oral care device for use in caring for the oral cavity; and a result outputter that outputs the determination result of the care mode to the oral care device.

Such an oral care mode determination device can detect the dental plaque amount and the poor condition level inside an oral cavity from an image of the oral cavity. If there is a large amount of dental plaque, cleaning performance should be enhanced. However, powerful cleaning performance is not suitable for a gingiva with a highly poor condition level. Since a proper care mode is determined from this viewpoint in accordance with both the dental plaque amount and the poor condition level, a proper care mode can be output as a determination result. Thus, it is possible to determine the care mode, for achieving proper cleaning performance, in which the oral care device (an oral cleaning device) is to be controlled. Then, the oral care device that has received the output of the determination result of the care mode can perform oral care under driving control defined by the care mode as the determination result.

In addition, an oral care mode determination device according to the second aspect of the present disclosure is the oral care mode determination device according to the first aspect, in which the image is an image captured by detecting light generated as a result of the oral cavity being irradiated with irradiation light of a predetermined wavelength to which dental plaque is responsive, and the dental plaque inside the oral cavity responding to the irradiation light.

As such, it is possible to detect the dental plaque amount by using the image captured by detecting the light generated as a result of the oral cavity being irradiated with the irradiation light of the predetermined wavelength to which dental plaque is responsive, and the dental plaque inside the oral cavity responding to the irradiation light. That is, it is possible to enhance the detection accuracy of the dental plaque amount.

In addition, an oral care mode determination device according to the third aspect of the present disclosure is the oral care mode determination device according to the first or second aspect, in which the image includes two or more images that include a first image showing a portion of the oral cavity and a second image showing an other portion of the oral cavity, the dental plaque adhesion amount detector detects the dental plaque amount for each of the portion and the other portion of the oral cavity, the poor-condition detector detects the poor condition level for each of the portion and the other portion of the oral cavity, the care mode determiner determines the care mode for each of the portion and the other portion of the oral cavity, and the result outputter outputs the determination result of the care mode for the portion of the oral cavity in association with the portion of the oral cavity, and outputs the determination result of the care mode for the other portion of the oral cavity in association with the other portion of the oral cavity.

As such, the detection of the dental plaque amount, the detection of the poor condition level, and the determination of the care mode can be performed individually for each of the portion and the other portion of the oral cavity. Thus, it is possible to output the proper care modes based on the dental plaque amounts and the poor condition levels of the portion and the other portion of the oral cavity.

In addition, an oral care mode determination device according to the fourth aspect of the present disclosure is the oral care mode determination device according to the third aspect, in which the result outputter outputs the determination result of the care mode for the portion of the oral cavity in association with a position inside the oral cavity corresponding to the portion of the oral cavity, and outputs the determination result of the care mode for the other portion of the oral cavity in association with a position inside the oral cavity corresponding to the other portion of the oral cavity.

As such, the output determination results of the care mode can be identified by the positions inside the oral cavity corresponding to the portion and the other portion of the oral cavity, and then the identified determination results can be used. That is, it is possible to obtain, from the positions inside the oral cavity corresponding to the portion and the other portion of the oral cavity, the determination results of the care mode associated with the positions. For instance, when the oral care device is used for cleaning a portion at a given position inside the oral cavity, it is possible to, for example, clean the portion in a proper care mode associated with the position.

In addition, an oral care mode determination device according to the fifth aspect of the present disclosure is the oral care mode determination device according to one of the first to fourth aspects, in which the care mode determiner determines the care mode by selecting a candidate from among a plurality of candidates, and each of the plurality of candidates for the care mode includes at least two types of control parameters each of which defines a different driving control of the oral care device, the at least two types of control parameters being set per candidate in the plurality of candidates.

As such, the two or more types of control parameters included in the determined care mode can define the driving controls of the oral care device.

In addition, an oral care mode determination device according to the sixth aspect of the present disclosure is the oral care mode determination device according to the fifth aspect, in which at least one type of control parameter among the at least two types of control parameters is a parameter for adjusting the tooth cleaning performance of the oral care device, and at least an other type of control parameter among the at least two types of control parameters is a parameter for complementing the tooth cleaning performance of the oral care device.

This makes it easier to balance the cleaning performance and suppression of the effects of deteriorating the poor condition level, by using the parameter for adjusting the tooth cleaning performance of the oral care device and the parameter for complementing the cleaning performance of the oral care device among the two or more types of control parameters included in the determined care mode.

In addition, an oral care mode determination device according to the seventh aspect of the present disclosure is the oral care mode determination device according to one of the first to sixth aspects, in which the oral care device is a liquid flow type oral cleaning device that ejects a cleaning liquid toward a tooth, the care mode includes one or more types of control parameters each of which defines a different driving control of the oral care device, the at least one type of control parameter is at least one of a flow rate, ejection pressure, an ejection time period, or an ejection frequency of the cleaning liquid, and the care mode determiner determines the care mode to cause, by adjusting the at least one type of control parameter included in the care mode, a load applied to the gingiva in ejecting the cleaning liquid to be smaller as the poor condition level deteriorates.

As such, in the liquid flow type oral cleaning device, the care mode can be determined to cause, by adjusting the at least one type of control parameter which is at least one of the flow rate, ejection pressure, ejection time period, or ejection frequency of the cleaning liquid, a load applied to the gingiva in ejecting the cleaning liquid to be smaller as the poor condition level deteriorates.

In addition, an oral care mode determination device according to the eighth aspect of the present disclosure is the oral care mode determination device according to one of the first to sixth aspects, in which the oral care device is an electrically powered toothbrush device that drives a brush provided to the oral care device, the care mode includes one or more types of control parameters each of which defines a different driving control of the oral care device, the at least one type of control parameter is at least one of a driving torque, a driving stroke, a driving speed, or a driving time period when driving the brush, and the care mode determiner determines the care mode to cause, by adjusting the at least one type of control parameter included in the care mode, a load applied to the gingiva in driving the brush to be smaller as the poor condition level deteriorates.

As such, in the electrically powered toothbrush device, the care mode can be determined to cause, by adjusting the at least one type of control parameter which is at least one of the driving torque, driving stroke, driving speed, or driving time period when driving the brush, a load applied to the gingiva in ejecting the cleaning liquid to be smaller as the poor condition level deteriorates.

In addition, an oral care mode determination device according to the ninth aspect of the present disclosure is the oral care mode determination device according to the eighth aspect, in which the oral care device includes: a brushing pressure detector that detects brushing pressure applied to the brush; and a brushing information notifier that notifies a user of feedback information regarding the brushing pressure, the brushing information notifier sets one or more thresholds for brushing pressure, and when the brushing pressure detected exceeds one threshold among the one or more thresholds, the brushing information notifier provides the user with a notification suggesting reducing the brushing pressure, the care mode further includes a parameter regarding the one or more thresholds, and the care mode determiner determines the care mode to cause, by adjusting the parameter regarding the one or more thresholds included in the care mode, the one or more thresholds to be smaller as the poor condition level deteriorates.

As such, it is possible to provide the user with a notification to avoid the brushing pressure not exceeding the set threshold, according to the determined care mode. As a result, it is possible to perform oral care with proper brushing pressure.

In addition, an oral care mode determination device according to the tenth aspect of the present disclosure is the oral care mode determination device according to one of the first to ninth aspects, in which the result outputter further transmits the determination result of the care mode to a terminal device connected to the oral care mode determination device, and the terminal device includes at least one of a display that displays the determination result of the care mode received from the result outputter or a notifier that notifies by sound the determination result of the care mode received from the result outputter.

As such, the terminal device that received the output of the determination result of the care mode can perform at least one of displaying or notifying of the determination result.

In addition, an oral care mode determination device according to the eleventh aspect of the present disclosure is the oral care mode determination device according to one of the first to tenth aspects, in which the poor condition level is scale information indicating, as a numerical value, the condition of a least one of swelling or bleeding of the gingiva.

As such, it is possible to detect the poor condition level indicating, as a numerical value, the condition of at least one of swelling or bleeding of the gingiva.

In addition, an oral care mode determination device according to the twelfth aspect of the present disclosure is the oral care mode determination device according to one of the first to eleventh aspects, in which the care mode is determined according to an oral health condition table in which the determination result of the care mode is associated with a combination of scale information indicating the dental plaque amount as a numerical value and scale information regarding the poor condition level indicating, as a numerical value, the condition of a least one of swelling or bleeding of the gingiva.

As such, it is possible to determine a care mode suitable for care in accordance with the oral health condition table.

In addition, an oral care mode determination device according to the thirteenth aspect of the present disclosure is the oral care mode determination device according to the twelfth aspect, in which the care mode determiner updates the oral health condition table according to at least one of a tendency of change in previous detection results of the dental plaque amount or a tendency of change in previous detection results of the poor condition level.

As such, the oral health condition table can be updated according to at least one of the tendency of the change in the previous detection results of the dental plaque amount or the tendency of the change in the previous detection results of the poor condition level. For instance, when there is the tendency that the care mode determined in the past according to the oral health condition table is not suitable for care in terms of at least one of the dental plaque amount or the poor condition level, the oral health condition table can be updated to be suitable for care.

In addition, an oral care mode determination device according to the fourteenth aspect of the present disclosure is the oral care mode determination device according to the thirteenth aspect, in which when the tendency of the change in the previous detection results of the dental plaque amount does not show a decrease in the dental plaque amount, the care mode determiner updates the oral health condition table to enhance care performance in a determination result.

As such, when there is the tendency that the care mode determined in the past according to the oral health condition table is not suitable for care in terms of the dental plaque amount, the oral health condition table can be updated to be suitable for care.

In addition, an oral care mode determination device according to the fifteenth aspect of the present disclosure is the oral care mode determination device according to the thirteenth or fourteenth aspect, in which when the tendency of the change in the previous detection results of the poor condition level shows a deterioration in the poor condition level, the care mode determiner updates the oral health condition table to decrease care performance in a determination result.

As such, when there is a tendency that the care mode determined in the past according to the oral health condition table is not suitable for care in terms of the poor condition level, the oral health condition table can be updated to be suitable for care.

In addition, an oral care mode determination method according to the sixteenth aspect is an oral care mode determination method performed by a computer. The oral care mode determination method includes: detecting, from an image of an oral cavity, a dental plaque amount that is the adhesion amount of dental plaque inside the oral cavity; detecting, from the image, a poor condition level that is the level of a poor condition of a gingiva inside the oral cavity; determining, according to the dental plaque amount detected and the poor condition level detected, a care mode that defines driving control of an oral care device for use in caring for the oral cavity; and outputting the determination result of the care mode to the oral care device.

As such, the oral care mode determination method can provide the same effects as the oral care mode determination device described above.

In addition, a program according to the seventeenth aspect of the present disclosure is a program for causing the computer to execute the above oral care mode determination method.

As such, the program can provide, with the use of the computer, the same effects as the oral care mode determination device described above.

An oral cleaning device according to another aspect of the present disclosure is an oral cleaning device that cleans a tooth by causing an ejected cleaning liquid to hit the tooth. The oral cleaning device includes: a cleaning liquid ejector that ejects the cleaning liquid from an ejection port in a predetermined direction; an ejector controller that controls at least one of the flow rate or the ejection pressure of the cleaning liquid ejected from the ejection port; a light irradiator that irradiates, with irradiation light, an irradiation area that includes a predetermined point on an extended line extending in the predetermined direction from the ejection port, and extends in a direction intersecting the extended line; and a light detector that detects detection light emitted in response to the irradiation light, in a two-dimensional detection area that includes the predetermined point and is included in the irradiation area. The ejector controller estimates, according to the result of detection by the light detector, a dental plaque portion within the detection area, determines, according to the estimation result of the dental plaque portion, the control amount of at least one of the flow rate or the ejection pressure, and causes the cleaning liquid to be ejected from the ejection port so as to match at least one of the flow rate or the ejection pressure to the determined control amount.

Such an oral cleaning device can match at least one of the flow rate or ejection pressure of the cleaning liquid to be ejected from the ejection port to the control amount determined according to the estimation result of the dental plaque portion. The estimation result of the dental plaque portion is the result of estimation by detecting, in the two-dimensional detection area, the detection light emitted in response to the irradiated irradiation light. For instance, when the irradiation light is light that can selectively make dental plaque emit a fluorescent color, it is possible to enhance the detection accuracy of the dental plaque portion by detecting, as detection light, light generated by making the dental plaque emit a fluorescent color. In this way, by properly estimating the dental plaque portion within the detection area, it is possible to determine the control amount, for the detection area, of at least one of the flow rate or the ejection pressure suitable for the estimated dental plaque portion. Thus, the oral cleaning device can clean the oral cavity at the properly controlled flow rate and ejection pressure of the cleaning liquid.

In addition, for instance, in still another aspect, in accordance with the detection result, the ejector controller may calculate the area of the dental plaque portion within the detection area from the area of a portion in which the detection light has been detected and that is included in the detection area, and the ejector controller may determine the control amount of at least one of the flow rate or ejection pressure of the cleaning liquid to be ejected from the ejection port to cause the control amount to be larger with an increase in the calculated area.

As such, by properly estimating the dental plaque portion within the detection area as the calculated area, it is possible to determine the control amount, for the detection area, of at least one of the flow rate or the ejection pressure suitable for the area of the estimated dental plaque portion. Thus, the oral cleaning device can clean the oral cavity at the properly controlled flow rate and ejection pressure of the cleaning liquid.

In addition, for instance, in yet another aspect, in accordance with the detection result, the ejector controller may calculate the adhesion amount of the dental plaque portion within the detection area from the luminance value of the detection light within the detection area, and the ejector controller may determine the control amount of at least one of the flow rate or ejection pressure of the cleaning liquid to be ejected from the ejection port to cause the control amount to be larger with an increase in the calculated adhesion amount.

As such, by properly estimating the dental plaque portion within the detection area as the adhesion amount calculated from the luminance value of the detection light, it is possible to determine the control amount, for the detection area, of at least one of the flow rate or the ejection pressure suitable for the estimated adhesion amount of the dental plaque portion. Thus, the oral cleaning device can clean the oral cavity at the properly controlled flow rate and ejection pressure of the cleaning liquid.

In addition, for instance, in yet another aspect, the irradiation light may include light of a wavelength that excites the metabolites of bacteria contained in the dental plaque portion, and the detection light may be fluorescence emitted by the excited metabolites.

As such, the irradiation light can excite the metabolites of bacteria contained in the dental plaque portion, and the fluorescence emitted by the excited metabolites can be detected as the detection light. Thus, by detecting the light selectively emitted by the dental plaque, it is possible to enhance the detection accuracy of the dental plaque portion.

In addition, for instance, in yet another aspect, the ejector controller may prohibit ejection of the cleaning liquid from the ejection port in in a prohibition period that partially overlaps a detection period from the start of detection of the detection light by the light detector until the end of the detection.

As such, although the ejected cleaning liquid tends to spread in droplets and this may affect the detection, the prohibition period in which ejection of the cleaning liquid is prohibited is provided, thereby creating a situation that makes it difficult for the cleaning liquid to spread in the prohibition period. In this way, the detection light can be detected.

In addition, for instance, in yet another aspect, the oral cleaning device may further include: a determiner that, when a state in which the value (brightness) of the detection light is less than or equal to a predetermined value has continued for a predetermined time or longer, determines that there is a blocking object blocking at least one of (i) the detection light between the detection area and the light detector or (ii) the irradiation light between the light irradiator and the irradiation area; and an outputter that outputs the determination result of the determiner.

As such, whether there is a blocking object blocking at least one of (i) the detection light between the detection area and the light detector or (ii) the irradiation light between the light irradiator and the irradiation area is determined by whether the state in which the value of the detection light is less than or equal to the predetermined value has continued for the predetermined time or longer, and a determination result can be output.

In addition, a control device according to yet another aspect of the present disclosure is a control device for an oral cleaning device that cleans a tooth by causing an ejected cleaning liquid to hit the tooth. The control device includes: an ejector controller that controls at least one of the flow rate or the ejection pressure of the cleaning liquid ejected from an ejection port for ejecting the cleaning liquid in a predetermined direction; an obtainer that obtains a result of detection of detection light emitted in response to irradiation light with which an irradiation area has been irradiated, the irradiation area including a predetermined point on an extended line extending in a predetermined direction from the ejection port, and extending in a direction intersecting the extended line, the detection light having been detected in a two-dimensional detection area included in the irradiation area and including the predetermined point. The ejector controller estimates a dental plaque portion within the detection area according to the obtained detection result, determines, according to the estimation result of the dental plaque portion, the control amount of at least one of the flow rate or the ejection pressure of the cleaning liquid to be ejected from the ejection port, and causes the cleaning liquid to be ejected from the ejection port so as to match the at least one of the flow rate or the ejection pressure to the determined control amount.

The control device can make the oral cleaning device using the control device have the same effects as the oral cleaning device described above.

In addition, a program according to yet another aspect of the present disclosure is a program for causing a computer to execute a control method for an oral cleaning device that cleans a tooth by causing an ejected cleaning liquid to hit the tooth. The control method includes: obtaining a detection result as a result of detecting detection light emitted in response to irradiation light with which an irradiation area has been irradiated, the irradiation area including a predetermined point on an extended line extending in a predetermined direction from an ejection port for ejecting the cleaning liquid in the predetermined direction, and extending in a direction intersecting the extended line, the detection light being detected in a two-dimensional detection area included in the irradiation area and including the predetermined point; estimating a dental plaque portion within the detection area according to the obtained detection result; determining, according to the estimation result of the dental plaque portion, the control amount of at least one of the flow rate or the ejection pressure of the cleaning liquid to be ejected from the ejection port; and causing the cleaning liquid to be ejected from the ejection port so as to match the at least one of the flow rate or the ejection pressure of the cleaning liquid to the determined control amount.

The program can provide, with the use of the computer, the same effects as the control device described above.

In addition, an oral cleaning method according to yet another aspect of the present disclosure is an oral cleaning method for cleaning a tooth by causing an ejected cleaning liquid to hit the tooth. The oral cleaning method includes: irradiating, with irradiation light, an irradiation area that includes a predetermined point on an extended line extending in a predetermined direction from an ejection port for ejecting the cleaning liquid in the predetermined direction, and extends in a direction intersecting the extended line; detecting detection light emitted in response to the emitted irradiation light in a two-dimensional detection area included in the irradiation area and including the predetermined point; estimating a dental plaque portion within the detection area according to the detection result of the detection light; determining the control amount of at least one of the flow rate or ejection pressure of the cleaning liquid to be ejected from the ejection port; and causing the cleaning liquid to be ejected from the ejection port so as to match the at least one of the flow rate or ejection pressure of the cleaning liquid to the determined control amount.

The oral cleaning method can provide the same effects as the oral cleaning device described above.

In addition, an oral cleaning device according to yet another aspect of the present disclosure is an oral cleaning device that cleans a tooth. The oral cleaning device includes a cleaning controller that controls cleaning performance that affects the tooth via a tooth cleaner; and a light detector that detects an image of a two-dimensional detection area including a cleaning effective area in which the tooth cleaner gives a cleaning effect. When the detected image includes a gingiva, the cleaning controller estimates the condition of the gingiva from the detected image, determines the control amount of cleaning performance in accordance with the estimation result of the condition of the gingiva, and controls the tooth cleaner so as to match the cleaning performance of the tooth cleaner to the determined control amount.

The oral cleaning device can match the cleaning performance to the control amount determined in accordance with the estimation result of the condition of the gingiva. The estimation result of the condition of the gingiva is the result of, when the image detected in the two-dimensional detection area includes a gingiva, estimation based on the detected image. In this way, by properly estimating the condition of the gingiva within the detection area, it is possible to determine the control amount, for the detection area, of the cleaning performance suitable for the estimated condition of the gingiva. Thus, the oral cleaning device can clean the oral cavity at the properly controlled flow rate and ejection pressure of the cleaning liquid.

In addition, for instance, an oral cleaning device according to yet another aspect is the oral cleaning device according to the first aspect, in which the tooth cleaner is a brush, and the cleaning controller includes a driving controller that controls the driving mode of the brush as the cleaning performance of the brush.

The oral cleaning device can set the driving mode of the brush to the driving mode determined in accordance with the estimation result of the condition of the gingiva. The estimation result of the condition of the gingiva is the result of, when the image detected in the two-dimensional detection area includes a gingiva, estimation based on the detected image. In this way, by properly estimating the condition of the gingiva within the detection area, it is possible to determine a driving mode, for the detection area, suitable for the estimated dental plaque portion. Thus, the oral cleaning device can clean the oral cavity in the properly controlled driving mode of the brush.

In addition, for instance, an oral cleaning device according to yet another aspect is the oral cleaning device according to the first aspect, in which the tooth cleaner is a liquid flow type cleaning liquid ejector that causes a cleaning liquid ejected from the ejection port toward the tooth to hit the tooth, and the cleaning controller functions as the ejector controller that controls at least one of the flow rate, ejection pressure, or ejection frequency of the cleaning liquid as the cleaning performance of the cleaning liquid ejector.

The oral cleaning device can match at least one of the flow rate, ejection pressure, or ejection frequency of the cleaning liquid ejected from the ejection port, to the control amount determined according to the estimation result of condition of the gingiva. The estimation result of the condition of the gingiva is the result of, when the image detected in the two-dimensional detection area includes a gingiva, estimation based on the detected image. In this way, by properly estimating the condition of the gingiva within the detection area, it is possible to determine the control amount, for the detection area, of at least one of the flow rate, the ejection pressure, or the ejection frequency suitable for the estimated condition of the gingiva. Thus, the oral cleaning device can clean the oral cavity at the properly controlled flow rate, ejection pressure, and ejection frequency of the cleaning liquid. By setting the ejection frequency to a proper ejection frequency suitable for the condition of the gingiva, that is to say, by performing proper intermittent ejection suitable for the condition of the gingiva, it gives the effect that can suppress a damage that will further deteriorate the condition of a gingiva with a poor condition.

In addition, for instance, an oral cleaning device according to yet another aspect is the oral cleaning device according to the third aspect, in which the estimation result indicates whether at least one of swelling or bleeding is found in the gingiva, and when the estimation result indicates that at least one of swelling or bleeding is found in the gingiva, the ejector controller determines the control amount of at least one of the flow rate, ejection pressure, or ejection frequency of the cleaning liquid to be ejected from the ejection port, to cause the control amount to be small compared with when the estimation result indicates that no swelling or bleeding is found in the gingiva.

As such, whether at least one of swelling or bleeding is found in gingiva is estimated as the estimated condition of the gingiva. When the estimation result indicates that at least one of swelling or bleeding is found in gingiva, the control amount of at least one of the flow rate, ejection pressure, ejection frequency of the cleaning liquid to be ejected from the ejection port can be determined to cause the control amount to be small compared with when the estimation result indicates that no swelling or bleeding is found in the gingiva. Thus, the oral cleaning device can clean the oral cavity at the properly controlled flow rate, ejection pressure, and ejection frequency of the cleaning liquid.

In addition, for instance, an oral cleaning device according to yet another aspect is the oral cleaning device according to the first or second aspect, in which the ejector controller prohibits ejection of the cleaning liquid from the ejection port in a prohibition period that partially overlaps a detection period from the start of detection of the detection light by the light detector until the end of the detection.

As such, although the ejected cleaning liquid tends to spread in droplets and this may affect the detection, the prohibition period in which ejection of the cleaning liquid is prohibited is provided, thereby creating a situation that makes it difficult for the cleaning liquid to spread in the prohibition period. In this way, the detection light can be detected.

In addition, for instance, an oral cleaning device according to yet another aspect is the oral cleaning device according to one of the first to fifth aspects, in which in accordance with the detection result of the light detector, the cleaning controller estimates the condition of the gingiva in a cleaning effective area that is a portion of the detection area and in which the tooth cleaner gives a cleaning effect, and the cleaning controller determines the control amount of cleaning performance in accordance with the estimation result of the condition of the gingiva.

In addition, a control device according to yet another aspect of the present disclosure is a control device for an oral cleaning device that cleans a tooth by using a tooth cleaner. The control device includes a cleaning controller that controls cleaning performance that affects the tooth via the tooth cleaner; and an image obtainer that obtains an image of a two-dimensional detection area including a cleaning effective area in which the tooth cleaner gives a cleaning effect. When the obtained image includes a gingiva, the cleaning controller estimates the condition of the gingiva from the obtained image, determines the control amount of the cleaning performance that will affect the tooth via the tooth cleaner, in accordance with the estimation result of the condition of the gingiva, and performs control so as to match the cleaning performance to the determined control amount.

The control device can make the oral cleaning device using the control device have the same effects as another oral cleaning device described above.

In addition, a program according to yet another aspect of the present disclosure is a program for causing a computer to execute a control method for an oral cleaning device that cleans a tooth by using a tooth cleaner. The control method includes: obtaining an image of a two-dimensional detection area including a cleaning effective area in which the tooth cleaner gives a cleaning effect; when the obtained image includes a gingiva, estimating the condition of the gingiva from the obtained image; determining the control amount of cleaning performance that will affect the tooth via the tooth cleaner, in accordance with the estimation result of the condition of the gingiva; and performing control so as to match the cleaning performance to the determined control amount.

The program can provide, with the use of the computer, the same effects as another control device described above.

**In addition,** an oral cleaning method according to yet another aspect of the present disclosure is an oral cleaning method using an oral cleaning device that cleans a tooth by using a tooth cleaner. The oral cleaning method includes: obtaining an image of a two-dimensional detection area including a cleaning effective area in which the tooth cleaner gives a cleaning effect; determining the control amount of cleaning performance that will affect the tooth via the tooth cleaner, in accordance with the detection result of the image; and performing control so as to match the cleaning performance to the determined control amount.

The oral cleaning method can provide the same effects as another oral cleaning device described above.

It should be noted that these general or specific aspects may be embodied as a system, a method, an integrated circuit, a computer program, or a computer-readable recording medium such as a CD-ROM or may be embodied as any combination of the system, the method, the integrated circuit, the computer program, and the recording medium.

It should be noted that the embodiment described below indicates a comprehensive or specific example. The numerical values, shapes, constituent elements, arrangement and connection of the constituent elements, steps, order of steps, and other details indicated in the embodiments described below are merely examples, and do not intend to limit the present disclosure. Moreover, the constituent elements not recited in the independent claims, among those described in the embodiment below are described as optional constituent elements.

Moreover, the figures are schematic illustrations and are not necessarily precise depictions. Accordingly, for instance, the scales used in the figures are not necessarily the same. Moreover, in the figures, substantially the same elements are assigned the same reference symbols, and overlapping explanations are omitted or simplified.

Moreover, in the specification, terms indicating relationships between elements such as parallel, identical, and orthogonal, terms indicating the shapes of elements such as ring-shaped, numerical values, and a numerical value range are not expressions indicating only strict meanings but expressions intended to include substantially equivalent ranges such as a difference of around several percentages (for example, around 10%).

### [Embodiment]

Hereinafter, an oral cleaning device according to an embodiment is described with reference to FIGS. 1 to 6.

### [1. Configuration of Oral Cleaning Device]

First, a configuration of the oral cleaning device according to the embodiment is described with reference to FIGS. 1 and 2. FIG. 1 illustrates a usage example of, for example, an oral cleaning device according to the embodiment. It should be noted that (a) in FIG. 1 illustrates oral cleaning device 100 in use, and (b) in FIG. 1 illustrates terminal device 200 connected to oral cleaning device 100 during the use of oral cleaning device 100. Moreover, (c) in FIG. 1 illustrates oral cleaning device 100c according to another example of the embodiment. In (c) in FIG. 1, some of the bristle bundles of brush 103b are not illustrated in order to enhance the visibility of the configuration of a portion that overlaps brush 103b. Moreover, FIG. 2 is a block diagram illustrating a functional configuration of, for example, the oral cleaning device according to the embodiment.

As illustrated in (a) in FIG. 1, oral cleaning device 100 is a liquid flow type cleaning device that cleans an oral cavity by ejecting a cleaning liquid toward the oral cavity of a user with the use of cleaning liquid ejector 103, which is an example of a tooth cleaner, to remove adhering things adhering to teeth 99 of the user. Alternatively, as illustrated in (c) in FIG. 1, oral cleaning device 100c can be achieved as an electrically powered toothbrush device intended to remove adhering things adhering to teeth 99 of the user and provided with brush 103b, which is an example of the tooth cleaner. While holding a handle not illustrated in (a) or (c) in FIG. 1, the user moves their hand holding the handle to change the position and orientation of ejection port 103a for ejecting the cleaning liquid or the position and orientation of brush 103b to be moved along the surfaces of teeth. While changing the position and orientation, the user causes the ejected cleaning liquid to hit a portion at a given position (e.g., tooth 99) inside the oral cavity or brings brush 103b into contact with the portion at the given position. The impact of the cleaning liquid hitting the portion can remove adhering things adhering to the portion the cleaning liquid hit. Moreover, brush 103b brought into contact with the portion reciprocates in a certain direction along the surfaces of the teeth in the vicinity of the portion, which can remove adhering things adhering to the brushing track of the brush. Such adhering things include food debris from eating and dental plaque 99a and a dental calculus containing bacteria which have grown with, for example, the food debris as media. Hereinafter, unless otherwise noted, an example of liquid flow type oral cleaning device 100 that ejects a cleaning liquid to remove adhering things adhering to the teeth of the user is described as an oral cleaning device.

In the embodiment, oral cleaning device 100 is configured in a manner that enables the user to visually identify the position of a portion the cleaning liquid hits. Specifically, oral cleaning device 100 includes camera 101, and an image captured by camera 101 detecting light inside an oral cavity is displayed on, for example, terminal display 203 of terminal device 200 of the user. This enables the user to clean their oral cavity while visually identifying the position of the portion that ejection port 103a is currently facing and toward which the cleaning liquid is being ejected.

**Incidentally,** since the colors of dental plaque 99a among adhering things are similar to the colors of teeth 99 (white to pale whites such as beige), it is difficult to visually identify dental plaque 99a by using only a normal camera. Thus, in oral cleaning device 100 in the embodiment, an image is generated in which the colors of dental plaque 99a are displayed in a manner that can distinguish from the colors of teeth 99 as illustrated in (b) in FIG. 1 by selectively making dental plaque 99a emit a fluorescent color. Furthermore, through analysis of the image, oral cleaning device 100 changes the ejection intensity of the cleaning liquid (at least one of the flow rate, the ejection pressure, or the ejection frequency of the cleaning liquid) when dental plaque 99a is positioned opposite ejection port 103a. In this way, oral cleaning device 100 can properly clean the oral cavity by ejecting the cleaning liquid at proper intensity (in other words, cleaning performance).

The cleaning performance should not be too powerful or too weak in terms of oral care. For instance, when the cleaning performance (care performance) is high, although removal of dental plaque is facilitated, damage to a gingiva tends to be large. Meanwhile, when the cleaning performance (care performance) is low, although it is difficult to remove the dental plaque, damage to the gingiva tends to be small. Thus, as for the cleaning performance (care performance) of proper cleaning of the oral cavity based on the condition of the gingiva, it is necessary to set the cleaning performance that is not too high or not too low. When determining that the cleaning performance in cleaning the oral cavity is too high or too low, a different criterion is set each time according to the amount of dental plaque adhering to teeth and the poor condition level of the gingiva. Thus, in the present disclosure, the settings of proper cleaning performance (care performance) in consideration of the poor condition level of a gingiva are explained in a variation of the embodiment.

Setting of the level of cleaning performance corresponds to selection of the driving mode of brush 103b in the example of oral cleaning device 100c. That is, oral cleaning device 100c is a toothbrush device. The driving modes of brush 103b include several driving modes based on the set values of, for example, a driving period and a driving torque for each of the following modes: a horizontal brushing mode in which brush 103b is moved in a direction in which a tooth and another adjacent tooth are arranged, and a beat brushing mode in which brush 103b is moved along the interval between a tooth and another adjacent tooth (that is, interdentium). That is, the intensity of the horizontal brushing mode and the intensity of the beat brushing mode are different between the driving modes of brush 103b. Thus, proper cleaning of the oral cavity is performed in the driving mode of proper intensity based on the adhesion position and the adhesion amount of dental plaque 99a. The driving modes include a beat-brushing-only driving mode with an intensity of the horizontal brushing mode of zero and a horizontal-brushing-only driving mode with an intensity of the beat brushing mode of zero. Alternatively, although not illustrated in the figures, an oral cleaning device with a rotary driven brush may also be considered as an example. Also in such a case, proper cleaning of the oral cavity can be performed in the driving mode of proper intensity.

Hereinafter, a detailed configuration of oral cleaning device 100 and a detailed configuration of terminal device 200 used in combination with oral cleaning device 100 are described with reference to FIGS. 1 and 2.

As illustrated in FIG. 2, oral cleaning device 100 includes camera 101, LED illuminator 102, cleaning liquid ejector 103, input acceptor 104, and control device 150. Camera 101, LED illuminator 102, cleaning liquid ejector 103, and input acceptor 104 are the hardware elements of oral cleaning device 100, and are also referred to as hardware. Meanwhile, control device 150 is a functional element that generates and outputs various control signals for controlling the operation of the hardware, and obtains information obtained as a result of the operation of the hardware. Control device 150 is achieved by executing a predetermined program using, for example, a computer, more specifically, a processor and memory. Thus, it can be said that the functional subject of control device 150 is the program to be executed using the processor and the memory.

It should be noted that in the explanations below, as illustrated in FIG. 2, an instance in which oral cleaning device 100 includes control device 150 inside is described. However, as long as control device 150 is configured in a manner that enables communication with the hardware, control device 150 may be disposed at any location. For instance, terminal device 200 can be internally provided with control device 150. A server device (not illustrated) such as an edge server or a cloud server can be internally provided with control device 150. If such a server device is internally provided with control device 150, the hardware and control device 150 are connected to each other via a network such as a wide area network or a local area (communication) network, thereby achieving an oral cleaning system as a combination of the hardware and control device 150 connected to each other via the network.

Control device 150 includes camera controller 151 connected to camera 101, LED illuminator controller 152 connected to LED illuminator 102, ejector controller 153 connected to cleaning liquid ejector 103, and memory 154 connected to input acceptor 104. Control device 150 further includes image processor 155 and dental plaque adhesion amount detector 156 that operate inside control device 150.

Camera 101 is an example of a light detector. As illustrated in FIG. 1, camera 101 is embodied as a combination of various optical elements, such as an image sensor, a lens, an aperture, and a filter. In the image sensor, a plurality of pixels are arranged in a matrix and detect the luminance values of the points of two-dimensional detection area 101a. Upon receiving an imaging start signal from camera controller 151, camera 101 starts image capturing. Upon receiving an imaging end signal from camera controller 151, camera 101 ends the image capturing. Then, camera 101 transmits a captured image to camera controller 151. The image here indicates the luminance value of each of red, green, and blue detected in each of the points of detection area 101a.

Moreover, detection area 101a contains a predetermined point on an extended line extending, from ejection port 103a, in the ejecting direction (also referred to as a predetermined direction) that is the direction in which a cleaning liquid is ejected. Then, the predetermined point corresponds to the distance between ejection port 103a and tooth 99 when an appropriate distance from tooth 99 is maintained in using oral cleaning device 100. Thus, the image of detection area 101a shows portions such as the surface of tooth 99 the cleaning liquid is hitting. For instance, in the figure, the thick dashed line arrow extending in the predetermined direction from ejection port 103a penetrates detection area 101a. Furthermore, detection area 101a has a thickness in a direction intersecting the plane in which detection area 101a extends. The thickness corresponds to the direction in which camera 101 receives light (the thin dashed line arrow in the figure), and includes an uneven spot such as a depression between two teeth 99 within detection area 101a.

Camera controller 151 controls image capturing by camera 101 and receives (obtains), from camera 101, the image obtained as a result of the image capturing. That is, camera controller 151 is an example of an obtainer. Then, camera controller 151 outputs the obtained image to image processor 155.

LED illuminator 102 is an example of a light irradiator. As illustrated in FIG. 1, LED illuminator 102 irradiates an irradiation area (a fan-shaped area between the two long dashed short dashed lines in FIG. 1) with irradiation light. Here, the irradiation area includes the predetermined point on the extended line extending in the ejecting direction from ejection port 103a, and extends in a direction intersecting the extended line. LED illuminator 102 is embodied by including, for example, an LED light source, a lens, and a power converter for driving the LED light source. LED illuminator 102 starts light irradiation upon receiving an irradiation start signal from LED illuminator controller 152, and ends the light irradiation upon receiving an irradiation end signal from LED illuminator controller 152. Light emitted from LED illuminator 102 (irradiation light) is a synthesized light including light that makes the outlines of, for example, teeth 99 and gingiva 98 visible on terminal device 200 and excitation light for selectively making dental plaque 99a emit a fluorescent color.

Here, dental plaque 99a consists of a clump of bacteria adhering to teeth 99 and the metabolites of the bacteria, and is also referred to as plaque. The metabolites include a type of substance that fluoresces due to excitation with light of a predetermined wavelength. In view of this, in the embodiment, irradiation light includes light of an excitation wavelength for exciting the metabolites and does not include a portion of light of a fluorescence wavelength which has undergone a wavelength shift. Thus, LED illuminator 102 includes an optical element capable of selecting a wavelength, such as a bandpass filter.

Examples of the above-mentioned metabolites include porphyrin. When irradiated with blue light of a wavelength of around 405 nm, porphyrin, which is a substance produced by bacteria contained in dental plaque 99a, fluoresces reddish purple to orange at a wavelength of around 660 nm. Since such fluorescence emission is not obtained from the enamel of the surfaces of teeth 99, it is possible to selectively make dental plaque 99a emit a fluorescent color. It should be noted that porphyrin is an example. Among the metabolites of the bacteria contained in dental plaque 99a, such a substance may be a substance in which the excitation wavelength and the fluorescence wavelength are sufficiently apart. When the wavelength of excitation light and the wavelength of fluorescence are different, it is possible to distinguish from mere reflected light that has reflected off the surface, which can selectively make dental plaque 99a emit a fluorescent color in the same manner as above.

It should be noted that porphyrin is accumulated according to the growth period of bacteria. Thus, porphyrin can also be used as an indication to measure how long adhering dental plaque 99a has been present. Camera 101 may be configured in a manner that can detect the fluorescence wavelength of fluorescence emitted from the metabolites.

Cleaning liquid ejector 103 includes, for example, an actuator for ejecting the cleaning liquid from ejection port 103a and a tank for storing the cleaning liquid. Cleaning liquid ejector 103 ejects the cleaning liquid from ejection port 103a at the flow rate and ejection pressure of the cleaning liquid determined by ejector controller 153. Thus, cleaning liquid ejector 103 receives, from ejector controller 153, a control signal specifying the flow rate and the ejection pressure, and ejects the cleaning liquid in accordance with the received control signal. However, the flow rate and ejection pressure of the cleaning liquid ejected from cleaning liquid ejector 103 changes according to the image of detection area 101a captured by camera 101. Thus, cleaning liquid ejector 103 receives a control signal specifying the flow rate and the ejection pressure from ejector controller 153 each time.

The cleaning liquid ejected by cleaning liquid ejector 103 may be, for example, just tap water and may be a chemical solution to enhance the cleaning effect and slurry containing, for example, fine particles for cleaning dental plaque 99a off.

Input acceptor 104 is a functional element that is included in, for example, a handle not illustrated in FIG. 1 and is for receiving input from the user. Input acceptor 104 is embodied as various sensors capable of detecting input from the user, such as a physical switch and a touch panel. Input acceptor 104 is used, for example, for the user themselves to input the set values of the flow rate and the ejection pressure of the cleaning liquid suitable for the user. Then, the input set values are stored via memory 154.

Memory 154 is a controller not illustrated in the figure that stores information in a storage device such as semiconductor memory and reads out the information where necessary. Thus, memory 154 has the function of accessing the storage device and storing the information in the storage device and the function of referring to and reading out the stored information.

It should be noted that terminal device 200 can also have functions similar to the functions of input acceptor 104. Specifically, as with input acceptor 104, for instance, terminal input acceptor 202 is used for the user themselves to input the set values of the flow rate and the ejection pressure of the cleaning liquid suitable for the user. Then, the input set values are stored via communicator 201 and memory 154.

By converting an image output from camera controller 151, image processor 155 improves the accuracy of estimation of the adhesion amount of dental plaque 99a in dental plaque adhesion amount detector 156, which is described later. Specifically, image processor 155 performs, on the image output from camera controller 151, processing for emphasizing the luminance near the fluorescence wavelength of a target metabolite. By doing so, it is possible to readily estimate, by a difference in the magnitude of the luminance value, a dental plaque portion to which dental plaque 99a is adhering within the image. Image processor 155 outputs the converted image to dental plaque adhesion amount detector 156.

Dental plaque adhesion amount detector 156 estimates the adhesion amount of dental plaque 99a in detection area 101a shown in the image. Then, the adhesion amount of dental plaque 99a estimated by dental plaque adhesion amount detector 156 is output to ejector controller 153 and is used in determining the flow rate and ejection pressure of the cleaning liquid. In this way, the adhesion amount of dental plaque 99a adhering to tooth 99 is estimated from the detected fluorescence, that is, the captured image. Then, the cleaning liquid can be ejected at proper flow rate and ejection pressure based on the estimated adhesion amount of dental plaque 99a. It should be noted that ejector controller 153 may include, as part of the functions thereof, image processor 155 and dental plaque adhesion amount detector 156. That is, ejector controller 153 may determine the flow rate and ejection pressure of the cleaning liquid directly from an image.

When detecting the adhesion amount of dental plaque 99a, dental plaque adhesion amount detector 156 calculates the area ratio of a portion to which dental plaque is adhering to the entirety of detection area 101a. When the area ratio is greater than or equal to a predetermined threshold, the cleaning liquid may be ejected at the flow rate and the ejection pressure for the case where dental plaque is adhering. Here, detection of the adhesion amount of dental plaque is described, focusing on ejection type oral cleaning device 100. Since ejection type oral cleaning device 100 ejects the cleaning liquid in the predetermined direction, the cleaning liquid hits the predetermined point on the extended line in the predetermined direction and the surrounding area thereof.

As such, cleaning effective area 101aa can be set that is narrower than detection area 101a and in which at least certain cleaning effects are expected (or simply, cleaning effects are obtained) as a result of the cleaning liquid hitting the area. When oral cleaning device 100 is embodied as a toothbrush device, cleaning effective area 101aa corresponds to a portion with which the brush came into contact in the track along which brush 103b was moved, in the toothbrush device held at a certain position when brush 103b was moved. Here, the following situation is assumed. The detection itself is performed before the brush comes into contact with a tooth. The toothbrush device is moved from the position where the detection was performed, in the extending direction of the brush (that is, toward the tooth) while keeping the orientation of the toothbrush device. The toothbrush device that has been moved there is held at the certain position.

In the embodiment, dental plaque adhesion amount detector 156 calculates the area ratio of the portion to which dental plaque is adhering to the entirety of cleaning effective area 101aa. When the area ratio is greater than or equal to a preset threshold, control is performed so as to eject the cleaning liquid at the flow rate and ejection pressure for the case where dental plaque is adhering. Thus, dental plaque adhesion amount detector 156 or image processor 155 has, for example, the function of trimming to extract, from detection area 101a, an image corresponding to cleaning effective area 101aa.

As described above, terminal device 200 is an information processing device of the user of oral cleaning device 100, such as a smartphone, a tablet terminal, and a personal computer, and can communicate with oral cleaning device 100 via communicator 201. Terminal device 200 includes communicator 201, terminal input acceptor 202, and terminal display 203. As the functions of the elements are described above, explanations for the functions are omitted here.

### [2. Operation of Oral Cleaning Device]

Then, the operation of oral cleaning device 100 configured as described above (an oral cleaning method) is described with reference to FIGS. 3 to 6. FIG. 3 is a flowchart illustrating an operation example of the oral cleaning device according to the embodiment. Moreover, FIG. 4 is a flowchart illustrating an example of an operation of the oral cleaning device according to the embodiment performed in a dental plaque detection ejection mode.

First, when oral cleaning device 100 starts operating, ejector controller 153 reads the normal ejection intensity of the cleaning liquid preset by the user (S101). For instance, ejector controller 153 inquires of memory 154, thereby causing memory 154 to read the normal ejection intensity stored as a set value in the storage device. Then, ejector controller 153 reads the normal ejection intensity via memory 154. It should be noted that the normal ejection intensity includes the flow rate and ejection pressure of the cleaning liquid, and is a value used as a default value when for instance dental plaque 99a is not adhering. It should be noted that if the set value of the normal ejection intensity is not stored in the storage device, the normal ejection intensity as an initial value is read, or a notification suggesting inputting the set value of the normal ejection intensity is output to the user.

Then, input acceptor 104 accepts, from the user, input regarding switching between on and off in order to switch on the dental plaque detection ejection mode (S102). Then, when no input has been received, that is, when it is determined that the dental plaque detection ejection mode is not on (No in S103), the cleaning liquid is constantly ejected at the normal ejection intensity (S104, the normal ejection intensity is also referred to as a normal ejection mode). Then, the processing ends.

Meanwhile, when input acceptor 104 accepts input to switch on the dental plaque detection ejection mode from the user, that is, when it is determined that the dental plaque detection ejection mode is on (Yes in S103), the cleaning liquid is ejected in the dental plaque detection ejection mode (S105), and the processing ends.

Step S105 in the above is described in detail with reference to FIG. 4. As illustrated in FIG. 4, when ejection in the dental plaque detection ejection mode starts due to Yes in step S103, ejector controller 153 reads the dental plaque detection ejection intensity of the cleaning liquid preset by the user (S201). For instance, ejector controller 153 inquires of memory 154, thereby causing memory 154 to read the dental plaque detection ejection intensity stored as a set value in the storage device. Then, ejector controller 153 reads the dental plaque detection ejection intensity via memory 154. It should be noted that the dental plaque detection ejection intensity includes the flow rate and ejection pressure of the cleaning liquid, and is used as a value when for instance dental plaque 99a is adhering. It should be noted that if the set value of the dental plaque detection ejection intensity is not stored in the storage device, the dental plaque detection ejection intensity as an initial value is read, or a notification suggesting inputting the set value of the dental plaque detection ejection intensity is output to the user.

Here, the normal ejection intensity and the dental plaque detection ejection intensity are explained with reference to FIG. 5. FIG. 5 is a graph illustrating a relationship between detection results and control amounts in the oral cleaning device according to the embodiment. As illustrated in FIG. 5, the control amount (both the flow rate and ejection pressure of the cleaning liquid are included in this case) at the dental plaque detection ejection intensity is larger than the control amount at the normal ejection intensity. Then, switching between the ejection intensities are performed on the basis of a threshold set for the area and adhesion amount of adhering dental plaque 99a. The area and adhesion amount of dental plaque 99a increase and exceed the threshold, the cleaning liquid is ejected at the dental plaque detection ejection intensity with the larger control amount.

That is, more intensive cleaning can be performed at the higher ejection intensity when the adhesion amount of dental plaque 99a is larger. Here, the area and adhesion amount of adhering dental plaque 99a are described. In the embodiment, the area of a dental plaque portion within detection area 101a that is a portion to which dental plaque 99a is adhering is used as the adhesion amount of dental plaque 99a. It can be estimated that the larger the area is, more dental plaque 99a is contained in the detection area.

Then, by ejecting the cleaning liquid at higher ejection intensity to remove a relatively large amount of adhering dental plaque 99a, it is possible to make the ejection intensity proper in terms of the efficiency of removal of dental plaque 99a. It should be noted that whether dental plaque 99a is adhering is determined by whether in each pixel in the captured image, the luminance of a color corresponding to the fluorescence wavelength exceeds a luminance threshold. When the number of pixels exceeding the luminance threshold exceeds a numerical value corresponding to the threshold illustrated in FIG. 5, the cleaning liquid is ejected at the flow rate and ejection pressure of the cleaning liquid at the dental plaque detection ejection intensity. It should be noted that since the luminance threshold may be affected by a condition such as pigmentation, the luminance threshold may be set for each user.

Moreover, as another method of calculating the adhesion amount of dental plaque 99a, the adhesion amount of dental plaque 99a within detection area 101a can be estimated by the total value of the luminance of the color corresponding to the fluorescence wavelength in each pixel within detection area 101a. The luminance of the color corresponding to the fluorescence wavelength in each pixel corresponds to the accumulation amount of metabolites at the actual position on the surface of tooth 99 corresponding to the pixel. Thus, the total value, for the entirety of detection area 101a, of the luminance of the color corresponding to the fluorescence wavelength is proportional to the adhesion amount of dental plaque 99a. As such, the adhesion amount of dental plaque 99a may be calculated in the above-mentioned manner. In this case, when the total value of the luminance exceeds the numerical value corresponding to the threshold illustrated in FIG. 5, cleaning liquid is ejected at the flow rate and ejection pressure of the cleaning liquid corresponding to the dental plaque detection intensity. Moreover, the control amount of the ejection intensity may be determined using both the area and the total luminance value in parallel.

FIG. 6 is another graph illustrating a relationship between detection results and control amounts in the oral cleaning device according to the embodiment. The example in FIG. 5 shows the relationship in which the ejection intensity sharply changes when the area and the total luminance value exceed the threshold. Meanwhile, for example, as illustrated in FIG. 6, the relationship between the area and total luminance value and the ejection intensity may be a continuously changing function. For instance, as illustrated in (a) in FIG. 6, a relationship similar to a logarithmic function may be applied in which the ejection intensity sharply increases in an interval where the area and the total luminance value are relatively small and the ejection intensity gently increases in an interval where the area and the total luminance value are relatively large. Moreover, for instance, as illustrated in (b) in FIG. 6, the relationship of a proportional function may be applied in which the ejection intensity increases in the same manner in both the interval where the area and the total luminance value are relatively small and the interval where the area and the total luminance value are relatively large. Moreover, for instance, as illustrated in (c) in FIG. 6, a relationship similar to an exponential function may be applied in which the ejection intensity gently increases in the interval where the area and the total luminance value are relatively small and the ejection intensity sharply increases in the interval where the area and the total luminance value are relatively large.

FIG. 4 is referenced again. After step S201, LED illuminator 102 irradiates the irradiation area with irradiation light including light of a wavelength that excites metabolites (S202). When the excited metabolites return to a ground state, fluorescence is generated from the dental plaque portion, of the irradiation area, to which dental plaque 99a is adhering, which selectively makes the dental plaque portion emit a fluorescent color.

Camera 101 detects the fluorescence as detection light within detection area 101a including the position on the extended line extending in the predetermined direction from ejection port 103a, that is, the position on the surface of tooth 99 in the ejecting direction of the cleaning liquid. That is, camera 101 captures an image including the fluorescence (S203). Since the detection here is performed in two-dimensional detection area 101a, it is possible to obtain the image, as described above, for which a comprehensive evaluation within detection area 101a is possible as with the area and the total luminance value. It should be noted that cleaning liquid ejector 103 may be prohibited from operating in order not to eject the cleaning liquid in a prohibition period that at least partially overlaps a detection period from the start of image capturing by camera 101 until the end of the image capturing. When the cleaning liquid is being ejected, the droplets of the cleaning liquid are spread inside the oral cavity. Thus, optical effects may occur. For this reason, as described above, it is effective to prohibit cleaning liquid ejector 103 from operating in order not to eject the cleaning liquid in the prohibition period.

When the area and the luminance value (the adhesion amount of dental plaque) are less than the threshold (No in S204), the cleaning liquid is ejected against detection area 101a at the normal ejection intensity (S205). Moreover, when the area and the luminance value are greater than or equal to the threshold (Yes in S204), the cleaning liquid is ejected against detection area 101a at the dental plaque detection ejection intensity (that is, high ejection intensity) (S206). After step S205 and step S206, the user moves their hand holding oral cleaning device 100, and performs the same operation on another detection area 101a. For this reason, whether the user has finished cleaning is determined (S207). For instance, when it is determined that the user has finished cleaning, according to, for example, the user's operation of pressing a finish button (not illustrated) (Yes in S207), the processing ends. When the user continues to clean (No in S207), the processing returns to step S202, and the same processing is repeated.

In this way, by making the metabolites of bacteria contained in dental plaque 99a fluoresce, thereby selectively making them emit colors, the dental plaque adhesion portion to which dental plaque 99a is adhering is selectively detected. The detection is performed on detection area 101a, which is a two-dimensional area. As a result, it is possible to eject the cleaning liquid against the portion, as an area, with a large adhesion amount of dental plaque 99a at a high ejection intensity (flow rate and ejection pressure). As such, it is possible to perform control for enabling proper ejection intensity in terms of removal of dental plaque 99a.

### [3. Variation 1 of Oral Cleaning Device]

Next, an oral cleaning device according to Variation 1 is described with reference to FIGS. 7A to 9. It should be noted that in Variation 1 described below, differences from the embodiment described above are stated, and explanations for substantially the same points in Variation 1 and the embodiment are omitted.

FIG. 7A is a block diagram illustrating a functional configuration of, for example, an oral cleaning device according to Variation 1 of the embodiment. As illustrated in FIG. 7A, as a difference from the embodiment, oral cleaning device 100a in Variation 1 includes poor-condition detector 157.

Poor-condition detector 157 estimates the condition of a gingiva, and performs control such that a cleaning liquid is ejected at ejection intensity suitable for the condition of the gingiva. Poor-condition detector 157 is connected to image processor 155, obtains a converted image, and estimates the condition of the gingiva from the converted image. It should be noted that poor-condition detector 157 may obtain a pre-conversion image from camera controller 151.

Poor-condition detector 157 includes a machine learning model that has been trained in advance and that has learned a correlation between a gingiva shown in an image and the presence or absence of a poor condition in the gingiva. By inputting the obtained image into the machine learning model, when a gingiva is shown in the image, poor-condition detector 157 can obtain the presence or absence of a poor condition in the gingiva as output. Here, the poor condition of the gingiva means, for example, occurrence of at least one of swelling or bleeding of the gingiva. In addition, the poor condition may include gingival conditions clinically considered as poor conditions. In this case, the machine learning model may be trained using, as labeled training data, an image showing a gingiva with the poor condition and ground truth data indicating the presence of the poor condition.

For instance, FIG. 7B illustrates an example of data for machine learning in the oral cleaning device according to Variation 1 of the embodiment. FIG. 7B illustrates intraoral images in two cases. FIG. 7B illustrates conditions such as "a gingiva has a red tinge (the hatched portion indicated by arrow (a) in the figure)", "an interdental gingiva is rounded (the portion indicated by arrow (b) in the figure)", "the root of a tooth is exposed (the portion indicated by arrow (c) in the figure)", "an interdental gingiva recedes (the portion indicated by arrow (d) in the figure)", "a gingiva is deformed (the portion indicated by arrow (e) in the figure)", and "a gingiva is bleeding (the hatched portion indicated by arrow (f) in the figure)". The machine learning model may be trained to learn each of the above conditions, using a data set including training image data items including a plurality of images showing similar conditions and ground truth data such as a probing value corresponding to each training data item and/or dentist's diagnosis data.

It should be noted that FIG. 7B illustrates the images in which the outer side of teeth (the anterior side of a subject, that is, the front of teeth) is observed. However, the presence or absence of a poor condition in the gingiva may be evaluated for each of the anterior side area and the posterior side area of each tooth, using also an image in which the inner side of teeth (the posterior side of the subject, that is, the back of teeth) is observed. Then, the machine learning model may be trained using a data set including training image data items including a plurality of images showing similar conditions and ground truth data such as the presence or absence of a poor condition in each training image data item, that is, a probing value and/or dentist's diagnosis data.

Moreover, there is no particular limitation for a correspondence between a poor gingival condition and teeth. The poor condition of a gingiva for one tooth, that is, for each tooth may be evaluated. The poor condition of a gingiva corresponding to a group of teeth (e.g., a maxillary teeth group, a mandibular teeth group, a posterior teeth group, and an anterior teeth group) may be evaluated.

If the cleaning liquid is ejected against detection area 101a including the gingiva with such a poor condition at high ejection intensity, the poor condition of the gingiva may deteriorate. For this reason, in Variation 1, when a poor condition is detected in a gingiva, by decreasing the ejection intensity of the cleaning liquid for detection area 101a (to, for example, ejection intensity lower than the normal ejection intensity), it is possible to suppress the poor condition of the gingiva from deteriorating. For instance, an evaluation result as described above may be converted into a score on a multi-point scale according to the level of poor condition of a gingiva. By doing so, it is possible to perform control such that the higher the score (the worse the poor condition level of the gingiva), the lower the ejection intensity and perform control such that the lower the score (the milder the poor condition level of the gingiva), the higher the ejection intensity. It should be noted that the number of score levels may correspond to the number of levels to which ejection intensity, that is, cleaning performance can be set. When the level of poor condition is evaluated using scores on a five-point scale, a cleaning performance among five cleaning performances is set, and one or more teeth can be cleaned with the set cleaning performance. As an example of a specific operation, oral cleaning device 100a operates in the following manner.

FIG. 8 is a flowchart illustrating an operation example of the oral cleaning device according to Variation 1 of the embodiment. Moreover, FIG. 9 is a flowchart illustrating an example of an operation of the oral cleaning device according to Variation 1 of the embodiment performed in both the dental plaque detection ejection mode and a poor-condition detection ejection mode.

As illustrated in FIG. 8, since step S301 and step S302 correspond to step S101 and step S102 in FIG. 3, explanations for these steps are omitted here. After step S302, input acceptor 104 further accepts, from the user, input regarding switching between on and off in order to switch on the poor-condition detection ejection mode (S303). Since step S304 corresponds to step S103 in FIG. 3, explanations for the step are omitted here. For No in step S304, the processing proceeds to step S305. For Yes in step S304, the processing proceeds to step S308. In both step S305 and step S308, whether input to switch on the poor-condition detection ejection mode has been received is determined.

For No in step S305, both the dental plaque detection ejection mode and the poor-condition detection ejection mode are off, the cleaning liquid is constantly ejected at the normal ejection intensity (S306, the normal ejection intensity is also referred to as the normal ejection mode). Then, the processing ends. For Yes in step S305, since the dental plaque detection ejection mode is off and the poor-condition detection ejection mode is on, the cleaning liquid is ejected in the poor-condition detection ejection mode (S307). Then, the processing ends.

For No in step S308, since the dental plaque detection ejection mode is on and the poor-condition detection ejection mode is off, the cleaning liquid is ejected in the dental plaque detection ejection mode (S309). Then, the processing ends. For Yes in step S308, since both the dental plaque detection ejection mode and the poor-condition detection ejection mode are on, the cleaning liquid is ejected in both the dental plaque detection ejection mode and the poor-condition detection ejection mode (S310). Then, the processing ends.

Step S310 in the above is described in detail with reference to FIG. 9. As illustrated in FIG. 9, after Yes in step S308, when ejection in both the dental plaque detection ejection mode and the poor-condition detection ejection mode starts, ejector controller 153 reads the dental plaque detection ejection intensity and the poor-condition detection ejection intensity of the cleaning liquid preset by the user (S201a). For instance, ejector controller 153 inquires of memory 154, thereby causing memory 154 to read the dental plaque detection ejection intensity and the poor-condition detection ejection intensity stored as set values in the storage device. Then, ejector controller 153 reads the dental plaque detection ejection intensity and the poor-condition detection ejection intensity via memory 154. It should be noted that the poor-condition detection ejection intensity includes the ejection frequency of the cleaning liquid in addition to the flow rate and ejection pressure of the cleaning liquid, and is a value used when for instance a gingiva within detection area 101a has a poor condition. By setting the ejection frequency to a proper ejection frequency suitable for a gingival condition, that is to say, by performing proper intermittent ejection suitable for the gingival condition, it gives the effect that can suppress a damage that will further deteriorate the condition of a gingiva with a poor condition. It should be noted that if the set value of the poor-condition detection ejection intensity is not stored in the storage device, the poor-condition detection ejection intensity as an initial value is read, or a notification suggesting inputting the set value of the poor-condition detection ejection intensity is output to the user.

Then, as with step S202 in FIG. 4, LED illuminator 102 irradiates an irradiation area with irradiation light including light of a wavelength that excites metabolites (S202a). Here, the irradiation light includes white light, which makes it possible to capture not only an image of fluorescence but also a normal image. By capturing an image of an oral cavity so as to include detection light in the image (S203a), it leads to a state in which a dental plaque portion selectively emits a fluorescent color, and when a gingiva is shown in detection area 101a, it is possible to obtain an image from which the condition of the gingiva can be estimated.

Then, determination in step S204 in FIG. 4 is performed. For No in step S204, the processing proceeds to step S401. For Yes in S204, the processing proceeds to step S402. In both step S401 and step S402, with regard to the estimated condition of the gingiva, whether a poor condition has been detected in the gingiva is determined. When it is determined that a poor condition has been detected in the gingiva (Yes in S401 or S402), the cleaning liquid is ejected against detection area 101a at the poor-condition detection ejection intensity (S403).

Since the other steps are similar to step S205 to step S207 in FIG. 4, explanations for these steps are omitted. Here, the example is described in which when a poor condition is detected in a gingiva, even if the adhesion amount of dental plaque 99a, that is, the area and the total luminance value are greater than or equal to the threshold, the cleaning liquid is ejected at the poor-condition detection ejection intensity. In this case, the poor-condition detection ejection mode is given priority over the dental plaque detection ejection mode. For instance, to give priority to the dental plaque detection ejection mode, when the result in step S204 is Yes, the processing may proceed to step S206 without performing step S402.

Moreover, in the above, the operation performed when both the dental plaque detection ejection mode and the poor-condition detection ejection mode are on is described. However, when the dental plaque detection ejection mode is off and the poor-condition detection ejection mode is on, steps S204, S402, and S206 in FIG. 9 are omitted. Thus, after step S203a, the processing proceeds to step S401 where whether a poor condition has been detected in the gingiva is determined. Then, the cleaning liquid is ejected at the normal ejection intensity or the poor-condition detection ejection intensity according to the determination result.

### [4. Variation 2 of Oral Cleaning Device]

Next, an oral cleaning device according to Variation 2 is described with reference to FIG. 10. It should be noted that in Variation 2 described below, differences from Variation 1 of the embodiment described above are stated, and explanations for substantially the same points in Variation 2 and Variation 1 of the embodiment are omitted.

FIG. 10 is a block diagram illustrating a functional configuration of, for example, an oral cleaning device according to Variation 2 of the embodiment. As a difference from oral cleaning device 100a according to Variation 1 described above, oral cleaning device 100b according to Variation 2 includes determiner 158 and outputter 159.

Determiner 158 and outputter 159 are, respectively, a functional element for determining whether light necessary to generate an image for use in determining the ejection intensity of a cleaning liquid reaches properly, and a functional element for notifying, if the light fails to reach, the failure. More specifically, determiner 158 obtains a converted image from the image processor. When a state in which the luminance of the entire image is low continues for a certain period of time or longer (a state in which the value (brightness) of the detected light is less than or equal to a predetermined value continues for the certain period of time or longer), determiner 158 determines that there is a blocking object that blocks at least one of (i) the detected light between detection area 101a and camera 101 or (ii) irradiation light between LED illuminator 102 and an irradiation area. Examples of such a blocking object include oral cavity structures such as lips, teeth, and gingivae, dust, and a foreign substance. When such a blocking object is present, detection of dental plaque and detection of a poor condition in a gingiva may not be performed properly. In oral cleaning device 100b according to Variation 2, outputter 159 outputs a determination result indicating the presence of the blocking object. For instance, a user is notified by displaying the determination result as an image on terminal display 203 of terminal device 200.

The user who has viewed the notification can respond to the situation so that various detections can be performed in a normal manner, by changing the orientation of oral cleaning device 100b and/or removing the blocking object, for instance.

### [5. Variation 3 of Oral Cleaning Device]

Next, an oral cleaning device according to Variation 3 is described with reference to FIGS. 11 to 13. It should be noted that in Variation 3 described below, differences from Variation 1 of the embodiment described above are states, and explanations for substantially the same points in Variation 3 and Variation 1 of the embodiment are omitted.

FIG. 11 is a flowchart illustrating an example of an operation of an oral cleaning device according to Variation 3 of the embodiment performed in both the dental plaque detection ejection mode and the poor-condition detection ejection mode. There are no differences in the functional configuration between the oral cleaning device according to Variation 3 and oral cleaning device 100a according to Variation 1 described above. Thus, here, the operation of the oral cleaning device according to Variation 3 is described with reference to FIG. 7A and the configuration of the oral cleaning device. It should be noted that since FIG. 11 and FIG. 9 are partially the same, explanations for common features may be omitted.

In the oral cleaning device according to Variation 3, dental plaque adhesion amount detector 156 indicates, on a discrete scale, the adhesion amount of dental plaque as a numerical value. Specifically, the adhesion amount of dental plaque is sorted into one of eight levels from 1 to 8 (the smaller the numerical value, the less the adhesion amount, and the larger the numerical value, the more the adhesion amount), and the numerical value corresponding to the level is output as the detection result of the adhesion amount. The estimation value of the adhesion amount used as the basis for detecting the adhesion amount is the same as the estimation value described in the embodiment. Thus, an explanation is omitted here. In this example, the adhesion amount is detected by replacing the estimation value of the adhesion amount with scale information. Moreover, in the oral cleaning device according to Variation 3, poor-condition detector 157 indicates, on a discrete scale, the level of poor condition (that is, poor condition level) of a gingiva as a numerical value. Specifically, the poor condition level of a gingiva is sorted into one of eight levels from 1 to 8 (the smaller the numerical value, the healthier the gingiva, the larger the numerical value, the worse the poor condition). Then, the numerical value corresponding to the level is output as the detection result of the poor condition level. The estimation value of the poor condition level used as the basis for detecting the poor condition level is the same as the conversion of the poor condition level into a score, which is described in Variation 1 of the embodiment. Thus, an explanation is omitted here. In this example, the poor condition level is detected by replacing the score of the poor condition level with scale information. It should be noted that the number of levels in the scale information stated above is just an example. The number of levels may be any number as long as the number is two or more.

Then, in Variation 3, using scale information on the dental plaque amount and scale information on the poor condition level, ejector controller 153 controls the cleaning performance used in the control of the oral cleaning device. Specifically, as a care mode that is an operation mode of the oral cleaning device, ejector controller 153 determines a proper care mode from the combination of the scale information on the dental plaque amount and the scale information on the poor condition level. Ejector controller 153 outputs control information indicating driving control to cause the oral cleaning device to be driven in the care mode obtained as a determination result. That is, care modes are for defining the driving controls of the oral cleaning device. Thus, ejector controller 153 in Variation 3 is a functional element having the functions of a care mode determiner and a result outputter and the function of a control signal generator that generates a control signal for the oral cleaning device.

Ejector controller 153 inquires of memory 154 in advance, thereby causing memory 154 to read an oral health condition table in which combinations of scale information on a dental plaque amount and scale information on a poor condition level are associated with care mode determination results, the oral health condition table being stored in a storage device. Then, ejector controller 153 reads the oral health condition table via memory 154 (S501). FIG. 12 illustrates an example of an oral health condition table according to a variation of the embodiment. As illustrated in FIG. 12, the care modes in the oral health condition table are care modes 1 to 4. One of the four care modes is output as the result of determination based on the combination of scale information on a dental plaque amount and scale information on a poor condition level. For instance, when the scale information on the dental plaque amount indicates 8 and the scale information on the poor condition level indicates 8, care mode 1 is output as a determination result.

As illustrated in FIG. 12, in the example, generally, the severity of the poor condition level is given priority in determining a care mode. Regardless of which of 1 to 6 is indicated in the scale information on the dental plaque amount, care mode 1 is output as a determination result when the scale information on the poor condition level indicates one of 5 to 8, care mode 2 is output as a determination result when the scale information on the poor condition level indicates 4, care mode 3 is output as a determination result when the scale information on the poor condition level indicates 2 or 3, and care mode 4 is output as a determination result when the scale information on the poor condition level indicates 1. It should be noted that with regard to 7 and 8 in the scale information on the dental plaque amount, care mode 1 is output as a determination result when the scale information on the poor condition level indicates one of 5 to 8, care mode 2 is output as a determination result when the scale information on the poor condition level indicates 4, care mode 3 is output as a determination result when the scale information on the poor condition level indicates 3, and care mode 4 is output as a determination result when the scale information on the poor condition level indicates 1 or 2.

Moreover, regarding the care modes, more various cleaning performances can be set. Specifically, as illustrated in FIG. 13, when an oral health condition table in another example is read, it is possible to control the oral cleaning device in a proper care mode determined from among various cleaning performance options (care modes). FIG. 13 illustrates an example of an oral health condition table according to another example of the variation of the embodiment. The oral cleaning device in the example can individually control the flow rate and ejection pressure of the flow of water to be ejected and the frequency of intermittent ejection of water. Specifically, a numerical value corresponding to x of care mode xy indicates the flow rate and ejection pressure of the flow of water to be ejected, and one of 1 to 6, that is, one of six sets of the flow rate and ejection pressure of the flow of water to be ejected can be set. Moreover, an alphabetical letter corresponding to y of care mode xy indicates the frequency of intermittent ejection of water, and one of four alphabetical letters a to d, that is, one of four frequencies of intermittent ejection of water can be set. Thus, in the oral health condition table in this example, one of care modes 1a to 6d, that is, 24 (6 × 4) care modes is output as a determination result. For instance, when the scale information on the dental plaque amount indicates 8 and the scale information on the poor condition level indicates 8, care mode 1c is output as a determination result.

In the example, when the scale information on the dental plaque amount indicates 1, the frequency of intermittent ejection of water is fixed to a, whereas the flow rate and the ejection pressure of the flow of water to be ejected change between 1 to 4 according to the poor condition level. Thus, the oral cleaning device may be controlled in a care mode to which two or more types of parameters (control parameters) are set. In this case, the degree of contribution of the scale information on the dental plaque amount and the degree of contribution of the scale information on the poor condition level to the two or more types of parameters may not be the same.

For instance, one of two types of parameters can be used as a parameter for adjusting the tooth cleaning performance of the oral cleaning device. Examples of parameters of this type include the flow rate of the cleaning liquid and the ejection pressure of the cleaning liquid. The flow rate and the ejection pressure of the cleaning liquid need be maintained at or above certain levels in order to maintain the cleaning performance.

Moreover, the other type of parameter can be used as a parameter for complementing the cleaning performance of the oral cleaning device. Examples of parameters of this type include an ejection time period (a length of time) and an ejection frequency. It is necessary to decrease the flow rate and the ejection pressure of the cleaning liquid to decrease a load to be applied to a gingiva in order not to deteriorate the poor condition level. At that time, the decreased cleaning performance can be complemented by increasing the ejection time period and the ejection frequency. That is, it is possible to complement the decreased cleaning performance by adjusting the ejection time period and the ejection frequency, while decreasing a load to be applied to the gingiva by adjusting the cleaning performance. In a similar example, in the case of the driving modes of brush 103b in oral cleaning device 100c, a numerical value corresponding to x of care mode xy indicates the intensity of a horizontal brushing mode. One of six intensities from 1 to 6 may be settable. An alphabetical letter corresponding to y of care mode xy indicates the intensity of a beat brushing mode. One of four intensities from a to d may be settable.

In this case, for instance, one type of parameter among two types of parameters can be used as a parameter for adjusting the tooth cleaning performance of oral cleaning device 100c (a toothbrush device). Examples of parameters of this type include the driving torque when driving brush 103b and the driving stroke when driving brush 103b. The driving torque and driving stroke when driving brush 103b need be maintained at or above certain levels in order to maintain the cleaning performance.

Moreover, the other type of parameter can be used as a parameter for complementing the cleaning performance of oral cleaning device 100c. Examples of parameters of this type include a driving speed and a driving time period. It is necessary to decrease the driving torque and driving stroke when driving brush 103b to decrease a load to be applied to a gingiva in order not to deteriorate the poor condition level. At that time, the decreased cleaning performance can be complemented by increasing the driving speed and the driving time period. That is, it is possible to complement the decreased cleaning performance by adjusting the driving speed and the driving time period, while decreasing a load to be applied to the gingiva by adjusting the cleaning performance. It should be noted that as a general usage method of oral cleaning device 100c (a toothbrush device), a user holding oral cleaning device 100c presses brush 103b against teeth. As such, the brushing pressure applied when the brush is pressed against the teeth also plays an important role in the cleaning performance and the poor condition level. The point is described later in Variation 5.

The degree of contribution of the scale information on the dental plaque amount and the degree of contribution of the scale information on the poor condition level are different for each type of parameter since effectiveness for dental plaque removal and effects on the poor condition level are different for each type of parameter (that is, the flow rate and ejection pressure of the flow of water to be ejected and the frequency of intermittent ejection of water or the intensity of the horizontal brushing mode and the intensity of the beat brushing mode).

It should be noted that in FIG. 13, the care modes are set using the two types of parameters (from the combinations of the numerical values from 1 to 6 and the alphabetical letters a to d). However, the care modes may be set using three or more types of parameters that include one or more other types of parameters. For instance, when the care modes are set using three types of parameters, the oral cleaning device may be configured in such a manner that partial cleaning functions contributing to the cleaning performance can be individually controlled using the three types of parameters corresponding to the flow rate of the flow of water to be ejected, the ejection pressure of the flow of water to be ejected, and the frequency of intermittent ejection of water, respectively.

FIG. 11 is referenced again. After reading the oral health condition table, irradiation light is emitted (S202a), and an image of an oral cavity is captured so as to include detection light in the image (S203a). Then, dental plaque adhesion amount detector 156 obtains the captured image of the oral cavity and detects an adhesion amount (S502). Moreover, poor-condition detector 157 obtains the captured image of the oral cavity and detects the poor condition level (S503). Steps S501, S502, and S503 need not be performed in the order stated, and the steps may be performed in order different from the order stated. Ejector controller 153 refers to the oral health condition table and determines a care mode, using the detected adhesion amount and poor condition level (S504). Then, ejector controller 153 outputs a determination result to the control signal generator, and controls the oral cleaning device in the determined care mode (S505).

In this way, the oral cavity can be cleaned in the proper care mode based on both the dental plaque adhesion amount and the poor condition level of the gingiva.

It should be noted that the oral health condition table is usually different for each user and is made for each user. Thus, an operation such as a user authentication operation, which is not illustrated, may be performed to read the oral health condition table of the user. Moreover, even if the user is the same user, the oral health condition table may become no longer suitable with an elapse of time. Thus, an operation to update the oral health condition table may be performed. Specifically, the oral health condition table may be updated according to at least one of the tendency of change in the previous detection results of the dental plaque amount or the tendency of change in the previous detection results of the poor condition level. In this case, for instance, when the tendency of the change in the previous detection results of the dental plaque amount does not show a decrease in the dental plaque amount, the oral health condition table is considered not suitable in terms of removal of dental plaque. Thus, the oral health condition table is updated to enhance the care performance in a determination result. For instance, the oral health condition table may be updated to cause each of the care modes to be replaced with a care mode with care performance of one level higher.

When the tendency of the change in the previous detection results of the poor condition level shows a deterioration of the poor condition level, the oral health condition table is considered not suitable in terms of maintaining the condition of the gingiva. Thus, the oral health condition table is updated to decrease the care performance in a determination result. For instance, the oral health condition table may be updated to cause each of the care modes to be replaced with a care mode with care performance of one level lower. Moreover, machine learning may be used in updating the oral health condition table.

### [6. Variation 4 of Oral Cleaning Device]

Next, an oral cleaning device according to Variation 4 is described with reference to FIG. 14. It should be noted that in Variation 4 described below, differences from Variation 3 of the embodiment described above are stated, and explanations for substantially the same points in Variation 4 and Variation3 of the embodiment are omitted.

FIG. 14 is a block diagram illustrating a functional configuration of an oral cleaning system according to Variation 4 of the embodiment. Oral cleaning system 100d according to Variation 4 is a system that achieves functions similar to those of the oral cleaning device in Variation 3, by using devices. There are no differences in actual operation from the oral cleaning device according to Variation 3 described above except that functional elements are divided into the devices. Since FIG. 14 is partially the same as FIG. 7A, explanations may be omitted. As illustrated in FIG. 14, as a difference from Variation 3 described above, some elements of the control device in Variation 3 are included in a terminal device in Variation 4. Specifically, control device 150 according to Variation 4 includes a processing part pertaining to input/output to/from hardware, whereas a functional part that performs, for example, data processing is included in terminal device 200. Specifically, terminal device 200 includes image processor 155, dental plaque adhesion amount detector 156, poor-condition detector 157, care mode determiner 153b (a part of ejector controller 153 in Variation 3), and result outputter 153c (a part of ejector controller 153 in Variation 3). Thus, it can be said that terminal device 200 in Variation 4 includes an oral care mode determination device. Terminal device 200 obtains an image from control device 150, which is an external device. Then, operation until the result of care mode determination using the image is output is the same as the operation described in Variation 3.

Then, result outputter 153c outputs the determination result to memory 154 of control device 150 via communicator 201. As a result, the determination result is stored in a storage device. Ejector controller 153a (a part of ejector controller 153 in Variation 3) reads out the determination result from the storage device via memory 154, and controls cleaning liquid ejector 103.

Meanwhile, in Variation 4, camera 101 and LED illuminator 102 are configured as an independent device (sensing device 800), and cleaning liquid ejector 103 and input acceptor 104 are configured as another independent device (individual cleaning device 900). That is, oral cleaning system 100d in Variation 4 includes the four devices. Communication performed between the devices in Variation 4 may be wireless communication or wired communication. Moreover, a communication system used in communication between the devices is not limited to a particular communication system.

In this way, the oral cleaning device can be achieved as the oral cleaning system including the groups of devices having similar functions. It should be noted that because of the configuration where terminal device 200 includes the oral care mode determination device, generated various information items (such as an intraoral image, the dental plaque adhesion amount, the poor condition level of a gingiva, and the determination result of a care mode) can be displayed as images in real time or notified by sound in real time. This can contribute to the enhancement of usability. The images may be displayed by terminal display 203 (an example of a display), and the sound notification may be performed by a terminal speaker (an example of a notifier), which is not illustrated. Moreover, by the oral cleaning device described above transmitting the generated various information items to terminal device 200 through communication, it is possible to display the generated various information items although the real-time property is decreased to a certain extent.

### [7. Variation 5 of Oral Cleaning Device]

Next, an oral cleaning device according to Variation 5 is described with reference to FIG. 15. It should be noted that in Variation 5 described below, differences especially from oral cleaning device 100c (a toothbrush device) in Variation 3 of the embodiment described above are stated, and explanations for substantially the same points in Variation 5 and Variation 3 of the embodiment are omitted.

FIG. 15 is a block diagram illustrating a functional configuration of an oral cleaning system according to Variation 5 of the embodiment. As a difference from oral cleaning device 100c of brush 103b slidable type, oral cleaning system 100e according to Variation 5 provides a notification in consideration of brushing pressure. Since FIG. 15 is partially the same as FIG. 10, explanations may be omitted. As illustrated in FIG. 15, in Variation 5, in oral cleaning system 100e, adhering things adhering to, for example, teeth are removed by pressing brush 103b against teeth and sliding brush 103b. Brush 103b is an electrically-powered brush. Motor 103c rotates with power supplied from a power supply (not illustrated). By converting the rotary driving into reciprocating driving, or using the rotary driving as is, brush 103b is driven in a reciprocating manner or in a rotary manner.

Due to the characteristics of pressing brush 103b against teeth and sliding brush 103b, the cleaning performance of oral cleaning system 100e and the force that deteriorates the poor condition level are determined by the brushing pressure that is pressing pressure applied to the teeth by brush 103b. In other words, it is necessary to apply proper brushing pressure in order not to deteriorate the poor condition level while ensuring proper cleaning performance.

Thus, in Variation 5, oral cleaning system 100e includes pressure detector 105 for detecting the brushing pressure. Pressure detector 105 is an example of a brushing pressure detector. Then, pressure detector 105 outputs the detected brushing pressure to outputter 159. In Variation 5, outputter 159 is an example of a brushing information notifier. Outputter 159 compares the detected brushing pressure and one or more preset thresholds, and outputs, to a user, a notification suggesting adjusting the brushing pressure. Specifically, when the detected brushing pressure exceeds one of the one or more thresholds, outputter 159 suggests that the user should decrease the brushing pressure. When the detected brushing pressure falls below one of the one or more thresholds, outputter 159 suggests that the user should increase the brushing pressure. The threshold for suggesting increasing the brushing pressure may not be the same as the threshold suggesting decreasing the brushing pressure. In this way, the user can press brush 103b against the teeth with proper brushing pressure within a certain range of pressure that a person can apply.

Here, the above thresholds may be set for each user according to the dental plaque amount (in other words, required cleaning performance) and the poor condition level of the user. Thus, in Variation 5, a care mode includes, as a parameter, the thresholds of the brushing pressure. By doing so, as illustrated in, for example, FIG. 13, in determining a care mode suitable for the dental plaque amount and the poor condition level, it is possible to make, in accordance with the thresholds included in the determined care mode, the user press brush 103b against their teeth with proper brushing pressure. Here, outputter 159 reads the threshold parameter included in the care mode, and compares the parameter and the detected brushing pressure, thereby achieving the above processing. The oral cleaning device, etc. according to the embodiment of the present disclosure are described above. However, the present disclosure is not limited to the embodiment.

For instance, in Variations 3 and 4 of the embodiment described above, the instance in which a care mode is determined from an image is described. When for instance an image includes the entirety of a user's oral cavity, one care mode can be obtained from the image according to the overall condition of the user's oral cavity. Meanwhile, although not described in detail, in Variations 3 and 4, a care mode can be determined for each portion of the user's oral cavity. The determination can be performed on a partial image for which the determination is to be performed, the partial image having been extracted from the image by, for example, trimming. For instance, images corresponding to the user's teeth are extracted by trimming. The dental plaque amount is detected from each of the images extracted through the trimming. The poor condition level of a gingiva near each tooth is detected. In this way, a care mode for each tooth can be determined. Alternatively, the dental plaque amount of a teeth group consisting of two or more of the user's teeth may be detected in the same manner. The poor condition level of a gingiva near the teeth group may be detected. In this way, a care mode for each teeth group may be determined.

As above, when images are two or more images including a first image showing a portion of the oral cavity and a second image showing another portion of the oral cavity, the dental plaque amount and the poor condition level are detected from each of the images, and a care mode for each portion of the oral cavity may be determined. However, when a plurality of care modes are output as determination results, information indicating which care mode corresponds to which tooth (teeth) or teeth group(s) is needed. That is, the result outputter need to output the determination result of a care mode for a portion of the oral cavity, in association with the portion of the oral cavity, and output the determination result of a care mode for a different portion of the oral cavity, in association with the different portion of the oral cavity. A portion and another portion of the oral cavity may be given identifiers identifying teeth inside the oral cavity, and identified by the identifiers. Alternatively, the positions of a portion and another portion of the oral cavity may be determined using, for example, a sensor, and identified in accordance with the determined positions inside the oral cavity. It should be noted that the first image and the second image may be different portions extracted from one image through, for example, trimming, may be two images captured by the same image sensor at different timings, and may be two images captured by different image sensors at the same timing. Moreover, the first image and the second image may be different portions of one image. That is, the above trimming processing does not have to involve outputting of an image extracted after actual trimming. The image may be input as it is. Then, the dental plaque amount and the poor condition level may be detected and a care mode may be determined, by selectively using the pixel values of only a portion of the image or the pixel values of only another portion of the image.

In addition, the processing units included in the oral cleaning device according to the embodiment are typically embodied as LSIs, which are integrated circuits. The processing units may be made as individual chips. A part or all of the processing units may be incorporated into one chip.

In addition, circuit integration may be achieved not only as an LSI but also as a dedicated circuit or a general-purpose processor. A field programmable gate array (FPGA), which can be programmed after manufacturing an LSI, or a reconfigurable processor in which the connections and settings of circuit cells inside an LSI are reconfigurable may be used.

In addition, in the embodiment and the variations thereof, each of the constituent elements may be configured as dedicated hardware or may be achieved by executing a software program suitable for the constituent element. The constituent element may be achieved by a program executer, such as a CPU or a processor, reading and executing a software program stored in a recording medium, such as a hard disk or semiconductor memory.

In addition, one aspect of the present disclosure may be achieved as, for example, an oral cleaning method performed by the oral cleaning device or a control method for the oral cleaning device. Moreover, another aspect of the present disclosure may be a computer program for causing a computer to execute the characteristic steps of the oral cleaning method or the control method.

In addition, each of the oral cleaning devices according to the embodiment and the variations thereof may be achieved as a single device or a plurality of devices. When the oral cleaning device is achieved as devices, the constituent elements of the oral cleaning device may be divided into the devices in any sorting method. For instance, the portable terminal may include the control device. Moreover, for instance, at least one of the functional elements of the control device may be achieved as the portable terminal or a server (for example, a cloud server) communicable with the portable terminal. When the oral cleaning device is achieved as the devices, a communication method between the devices is not limited to a particular method, and the communication may be wireless or wired communication. Moreover, wireless communication and wired communication may be combined as communication between the devices. It should be noted that the cloud server may be a server capable of communicating with the portable terminal via, for example, the internet, and provide the portable terminal with an application for using an intraoral camera. For instance, a user downloads the application from the cloud server and installs the application on the portable terminal. Moreover, the cloud server may obtain, via the portable terminal, an image captured by the oral cleaning device.

In addition, the divided functional blocks illustrated in each block diagram are a mere example. Two or more functional blocks may be incorporated into one functional block. One functional block may be divided into more than one functional block. A part of the function may be transferred from one functional block to another functional block. Moreover, the same hardware or software may process the functions of two or more functional blocks having similar functions in parallel or on a time-sharing basis.

In addition, the order in which the steps in each flowchart are performed is provided as an example to specifically explain the present disclosure. The steps may be performed in order different from the above order. Moreover, one or more of the steps may be performed simultaneously (in parallel) with another step.

The present disclosure is not limited to the above one or more aspects. The scope of the one or more aspects of the present disclosure may also encompass embodiments obtained by adding, to the embodiment, various modifications envisioned by those skilled in the art, and embodiments constructed by combining constituent elements in different embodiments, as long as the resultant embodiments do not depart from the scope of the present disclosure.

### [Industrial Applicability]

The present disclosure is applicable to oral cleaning devices.

### [Reference Signs List]

98 gingiva
99 tooth, teeth
99a dental plaque
100, 100a, 100b, 100c oral cleaning device
100d oral cleaning system
101 camera
101a detection area
101aa cleaning effective area
102 LED illuminator
103 cleaning liquid ejector
103a ejection port
103b brush
104 input acceptor
150 control device
151 camera controller
152 LED illuminator controller
153, 153a ejector controller
153b care mode determiner
153c result outputter
154 memory
155 image processor
156 dental plaque adhesion amount detector
157 poor-condition detector
158 determiner
159 outputter
200 terminal device
201 communicator
202 terminal input acceptor
203 terminal display
800 sensing device
900 individual cleaning device

## Claims

1. An oral care mode determination device comprising:
a dental plaque adhesion amount detector (156) that detects, from an image of an oral cavity, a dental plaque amount that is an adhesion amount of dental plaque (99a) inside the oral cavity;
a poor-condition detector (157) that detects, from the image, a poor condition level that is a level of a poor condition of a gingiva inside the oral cavity;
a care mode determiner (153b) that determines, according to the dental plaque amount detected and the poor condition level detected, a care mode that defines driving control of an oral care device (100) for use in caring for the oral cavity; and
a result outputter (153c) that outputs a determination result of the care mode to the oral care device (100).

2. The oral care mode determination device according to claim 1, wherein
the image is an image captured by detecting light generated as a result of the oral cavity being irradiated with irradiation light of a predetermined wavelength to which dental plaque is responsive, and the dental plaque inside the oral cavity responding to the irradiation light.

3. The oral care mode determination device according to claim 1, wherein
the image includes two or more images that include a first image showing a portion of the oral cavity and a second image showing an other portion of the oral cavity,
the dental plaque adhesion amount detector detects the dental plaque amount for each of the portion and the other portion of the oral cavity,
the poor-condition detector detects the poor condition level for each of the portion and the other portion of the oral cavity,
the care mode determiner determines the care mode for each of the portion and the other portion of the oral cavity, and
the result outputter
outputs a determination result of the care mode for the portion of the oral cavity in association with the portion of the oral cavity, and
outputs a determination result of the care mode for the other portion of the oral cavity in association with the other portion of the oral cavity.

4. The oral care mode determination device according to claim 3, wherein
the result outputter outputs the determination result of the care mode for the portion of the oral cavity in association with a position inside the oral cavity corresponding to the portion of the oral cavity, and outputs the determination result of the care mode for the other portion of the oral cavity in association with a position inside the oral cavity corresponding to the other portion of the oral cavity.

5. The oral care mode determination device according to claim 1, wherein
the care mode determiner determines the care mode by selecting a candidate from among a plurality of candidates, and
each of the plurality of candidates for the care mode includes at least two types of control parameters each of which defines a different driving control of the oral care device, the at least two types of control parameters being set per candidate in the plurality of candidates.

6. The oral care mode determination device according to claim 5, wherein
at least one type of control parameter among the at least two types of control parameters is a parameter for adjusting tooth cleaning performance of the oral care device, and at least an other type of control parameter among the at least two types of control parameters is a parameter for complementing the tooth cleaning performance of the oral care device.

7. The oral care mode determination device according to claim 1, wherein
the oral care device is a liquid flow type oral cleaning device that ejects a cleaning liquid toward a tooth,
the care mode includes one or more types of control parameters each of which defines a different driving control of the oral care device,
the at least one type of control parameter is at least one of a flow rate, ejection pressure, an ejection time period, or an ejection frequency of the cleaning liquid, and
the care mode determiner determines the care mode to cause, by adjusting the at least one type of control parameter included in the care mode, a load applied to the gingiva in ejecting the cleaning liquid to be smaller as the poor condition level deteriorates.

8. The oral care mode determination device according to claim 1, wherein
the oral care device is an electrically powered toothbrush device that drives a brush provided to the oral care device,
the care mode includes one or more types of control parameters each of which defines a different driving control of the oral care device,
the at least one type of control parameter is at least one of a driving torque, a driving stroke, a driving speed, or a driving time period when driving the brush, and
the care mode determiner determines the care mode to cause, by adjusting the at least one type of control parameter included in the care mode, a load applied to the gingiva in driving the brush to be smaller as the poor condition level deteriorates.

9. The oral care mode determination device according to claim 8, wherein the oral care device includes:
a brushing pressure detector that detects brushing pressure applied to the brush; and
a brushing information notifier that notifies a user of feedback information regarding the brushing pressure,
the brushing information notifier sets one or more thresholds for brushing pressure, and when the brushing pressure detected exceeds one threshold among the one or more thresholds, the brushing information notifier provides the user with a notification suggesting reducing the brushing pressure,
the care mode further includes a parameter regarding the one or more thresholds, and
the care mode determiner determines the care mode to cause, by adjusting the parameter regarding the one or more thresholds included in the care mode, the one or more thresholds to be smaller as the poor condition level deteriorates.

10. The oral care mode determination device according to claim 1, wherein
the result outputter further transmits the determination result of the care mode to a terminal device connected to the oral care mode determination device, and
the terminal device includes at least one of a display that displays the determination result of the care mode received from the result outputter or a notifier that notifies by sound the determination result of the care mode received from the result outputter.

11. The oral care mode determination device according to any one of claims 1 to 10, wherein
the poor condition level is scale information indicating, as a numerical value, a condition of a least one of swelling or bleeding of the gingiva.

12. The oral care mode determination device according to any one of claims 1 to 10, wherein
the care mode is determined according to an oral health condition table in which the determination result of the care mode is associated with a combination of scale information indicating the dental plaque amount as a numerical value and scale information regarding the poor condition level indicating, as a numerical value, a condition of a least one of swelling or bleeding of the gingiva.

13. The oral care mode determination device according to claim 12, wherein
when the tendency of the change in the previous detection results of the poor condition level shows a deterioration in the poor condition level, the care mode determiner updates the oral health condition table to decrease care performance in a determination result.

14. An oral care mode determination method performed by a computer, the oral care mode determination method comprising:
detecting, from an image of an oral cavity, a dental plaque amount that is an adhesion amount of dental plaque (99a) inside the oral cavity;
detecting, from the image, a poor condition level that is a level of a poor condition of a gingiva (98) inside the oral cavity;
determining, according to the dental plaque amount detected and the poor condition level detected, a care mode that defines driving control of an oral care device for use in caring for the oral cavity; and
outputting a determination result of the care mode to the oral care device (100).

15. A program for causing the computer to execute the oral care mode determination method according to claim 14.

## Patentansprüche

1. Vorrichtung zur Bestimmung eines Mundpflegemodus, umfassend:
einen Detektor für die Anhaftungsmenge von Zahnbelag (156), der aus einem Bild einer Mundhöhle eine Zahnbelagmenge detektiert, die eine Anhaftungsmenge von Zahnbelag (99a) im Inneren der Mundhöhle ist;
einen Detektor für einen schlechten Zustand (157), der aus dem Bild einen Grad eines schlechten Zustands detektiert, der ein Grad eines schlechten Zustands eines Zahnfleisches im Inneren der Mundhöhle ist;
einen Bestimmer für einen Pflegemodus (153b), der gemäß der detektierten Zahnbelagmenge und dem detektierten Grad des schlechten Zustands einen Pflegemodus bestimmt, der eine Antriebssteuerung einer Mundpflegevorrichtung (100) zur Verwendung bei der Pflege der Mundhöhle definiert; und
einen Ergebnisausgeber (153c), der ein Bestimmungsergebnis des Pflegemodus an die Mundpflegevorrichtung (100) ausgibt.

2. Vorrichtung zur Bestimmung eines Mundpflegemodus nach Anspruch 1, wobei
das Bild ein Bild ist, das durch Detektieren von Licht aufgenommen wird, das dadurch erzeugt wird, dass die Mundhöhle mit Bestrahlungslicht einer vorbestimmten Wellenlänge bestrahlt wird, auf die Zahnbelag anspricht, und dass der Zahnbelag im Inneren der Mundhöhle auf das Bestrahlungslicht anspricht.

3. Vorrichtung zur Bestimmung eines Mundpflegemodus nach Anspruch 1, wobei
das Bild zwei oder mehr Bilder umfasst, die ein erstes Bild, das einen Abschnitt der Mundhöhle zeigt, und ein zweites Bild, das einen anderen Abschnitt der Mundhöhle zeigt, umfassen,
der Detektor für die Anhaftungsmenge von Zahnbelag die Zahnbelagmenge für jeden von dem Abschnitt und dem anderen Abschnitt der Mundhöhle detektiert,
der Detektor für einen schlechten Zustand den Grad des schlechten Zustands für jeden von dem Abschnitt und dem anderen Abschnitt der Mundhöhle detektiert,
der Bestimmer für einen Pflegemodus den Pflegemodus für jeden von dem Abschnitt und dem anderen Abschnitt der Mundhöhle bestimmt, und
der Ergebnisausgeber
ein Bestimmungsergebnis des Pflegemodus für den Abschnitt der Mundhöhle in Zuordnung zu dem Abschnitt der Mundhöhle ausgibt, und
ein Bestimmungsergebnis des Pflegemodus für den anderen Abschnitt der Mundhöhle in Zuordnung zu dem anderen Abschnitt der Mundhöhle ausgibt.

4. Vorrichtung zur Bestimmung eines Mundpflegemodus nach Anspruch 3, wobei
der Ergebnisausgeber das Bestimmungsergebnis des Pflegemodus für den Abschnitt der Mundhöhle in Zuordnung zu einer Position im Inneren der Mundhöhle, die dem Abschnitt der Mundhöhle entspricht, ausgibt, und das Bestimmungsergebnis des Pflegemodus für den anderen Abschnitt der Mundhöhle in Zuordnung zu einer Position im Inneren der Mundhöhle, die dem anderen Abschnitt der Mundhöhle entspricht, ausgibt.

5. Vorrichtung zur Bestimmung eines Mundpflegemodus nach Anspruch 1, wobei
der Bestimmer für einen Pflegemodus den Pflegemodus durch Auswählen eines Kandidaten aus einer Mehrzahl von Kandidaten bestimmt, und
jeder der Mehrzahl von Kandidaten für den Pflegemodus mindestens zwei Arten von Steuerungsparametern umfasst, von denen jede eine unterschiedliche Antriebssteuerung der Mundpflegevorrichtung definiert, wobei die mindestens zwei Arten von Steuerungsparametern pro Kandidat in der Mehrzahl von Kandidaten festgelegt sind.

6. Vorrichtung zur Bestimmung eines Mundpflegemodus nach Anspruch 5, wobei
mindestens eine Art von Steuerungsparameter unter den mindestens zwei Arten von Steuerungsparametern ein Parameter zum Einstellen einer Zahnreinigungsleistung der Mundpflegevorrichtung ist, und mindestens eine andere Art von Steuerungsparameter unter den mindestens zwei Arten von Steuerungsparametern ein Parameter zum Ergänzen der Zahnreinigungsleistung der Mundpflegevorrichtung ist.

7. Vorrichtung zur Bestimmung eines Mundpflegemodus nach Anspruch 1, wobei
die Mundpflegevorrichtung eine Mundreinigungsvorrichtung vom Flüssigkeitsströmungstyp ist, die eine Reinigungsflüssigkeit zu einem Zahn ausstößt,
der Pflegemodus eine oder mehrere Arten von Steuerungsparametern umfasst, von denen jede eine unterschiedliche Antriebssteuerung der Mundpflegevorrichtung definiert,
die mindestens eine Art von Steuerungsparameter mindestens eines von einer Durchflussrate, einem Ausstoßdruck, einer Ausstoßzeitdauer oder einer Ausstoßfrequenz der Reinigungsflüssigkeit ist, und
der Bestimmer für einen Pflegemodus den Pflegemodus so bestimmt, dass durch Einstellen der mindestens einen Art von Steuerungsparameter, die in dem Pflegemodus enthalten ist, eine auf das Zahnfleisch beim Ausstoßen der Reinigungsflüssigkeit ausgeübte Last kleiner wird, je mehr sich der Grad des schlechten Zustands verschlechtert.

8. Vorrichtung zur Bestimmung eines Mundpflegemodus nach Anspruch 1, wobei
die Mundpflegevorrichtung eine elektrisch betriebene Zahnbürstenvorrichtung ist, die eine an der Mundpflegevorrichtung vorgesehene Bürste antreibt,
der Pflegemodus eine oder mehrere Arten von Steuerungsparametern umfasst, von denen jede eine unterschiedliche Antriebssteuerung der Mundpflegevorrichtung definiert,
die mindestens eine Art von Steuerungsparameter mindestens eines von einem Antriebsdrehmoment, einem Antriebshub, einer Antriebsgeschwindigkeit oder einer Antriebszeitdauer beim Antreiben der Bürste ist, und
der Bestimmer für einen Pflegemodus den Pflegemodus so bestimmt, dass durch Einstellen der mindestens einen Art von Steuerungsparameter, die in dem Pflegemodus enthalten ist, eine auf das Zahnfleisch beim Antreiben der Bürste ausgeübte Last kleiner wird, je mehr sich der Grad des schlechten Zustands verschlechtert.

9. Vorrichtung zur Bestimmung eines Mundpflegemodus nach Anspruch 8, wobei die Mundpflegevorrichtung umfasst:
einen Bürstdruckdetektor, der einen auf die Bürste ausgeübten Bürstdruck detektiert; und
einen Bürstinformations-Benachrichtiger, der einen Benutzer über Rückmeldungsinformationen bezüglich des Bürstdrucks benachrichtigt,
wobei der Bürstinformations-Benachrichtiger einen oder mehrere Schwellenwerte für den Bürstdruck festlegt, und wenn der detektierte Bürstdruck einen Schwellenwert unter dem einen oder den mehreren Schwellenwerten überschreitet, der Bürstinformations-Benachrichtiger dem Benutzer eine Benachrichtigung bereitstellt, die vorschlägt, den Bürstdruck zu verringern,
der Pflegemodus ferner einen Parameter bezüglich des einen oder der mehreren Schwellenwerte umfasst, und
der Bestimmer für einen Pflegemodus den Pflegemodus so bestimmt, dass durch Einstellen des Parameters bezüglich des einen oder der mehreren Schwellenwerte, der in dem Pflegemodus enthalten ist, der eine oder die mehreren Schwellenwerte kleiner werden, je mehr sich der Grad des schlechten Zustands verschlechtert.

10. Vorrichtung zur Bestimmung eines Mundpflegemodus nach Anspruch 1, wobei
der Ergebnisausgeber das Bestimmungsergebnis des Pflegemodus ferner an ein Endgerät überträgt, das mit der Vorrichtung zur Bestimmung eines Mundpflegemodus verbunden ist, und
das Endgerät mindestens eines von einer Anzeige, die das von dem Ergebnisausgeber empfangene Bestimmungsergebnis des Pflegemodus anzeigt, oder einem Benachrichtiger, der das von dem Ergebnisausgeber empfangene Bestimmungsergebnis des Pflegemodus durch Ton benachrichtigt, umfasst.

11. Vorrichtung zur Bestimmung eines Mundpflegemodus nach einem der Ansprüche 1 bis 10, wobei
der Grad des schlechten Zustands eine Skaleninformation ist, die als Zahlenwert einen Zustand von mindestens einem von einer Schwellung oder einer Blutung des Zahnfleisches angibt.

12. Vorrichtung zur Bestimmung eines Mundpflegemodus nach einem der Ansprüche 1 bis 10, wobei
der Pflegemodus gemäß einer Mundgesundheitszustandstabelle bestimmt wird, in der das Bestimmungsergebnis des Pflegemodus einer Kombination aus einer Skaleninformation, die die Zahnbelagmenge als Zahlenwert angibt, und einer Skaleninformation bezüglich des Grades des schlechten Zustands, die als Zahlenwert einen Zustand von mindestens einem von einer Schwellung oder einer Blutung des Zahnfleisches angibt, zugeordnet ist.

13. Vorrichtung zur Bestimmung eines Mundpflegemodus nach Anspruch 12, wobei
wenn die Tendenz der Änderung in den vorherigen Detektionsergebnissen des Grades des schlechten Zustands eine Verschlechterung des Grades des schlechten Zustands zeigt, der Bestimmer für einen Pflegemodus die
Mundgesundheitszustandstabelle aktualisiert, um eine Pflegeleistung in einem Bestimmungsergebnis zu verringern.

14. Verfahren zur Bestimmung eines Mundpflegemodus, das von einem Computer ausgeführt wird, wobei das Verfahren zur Bestimmung eines Mundpflegemodus umfasst:
Detektieren, aus einem Bild einer Mundhöhle, einer Zahnbelagmenge, die eine Anhaftungsmenge von Zahnbelag (99a) im Inneren der Mundhöhle ist;
Detektieren, aus dem Bild, eines Grades eines schlechten Zustands, der ein Grad eines schlechten Zustands eines Zahnfleisches (98) im Inneren der Mundhöhle ist;
Bestimmen, gemäß der detektierten Zahnbelagmenge und dem detektierten Grad des schlechten Zustands, eines Pflegemodus, der eine Antriebssteuerung einer Mundpflegevorrichtung zur Verwendung bei der Pflege der Mundhöhle definiert; und
Ausgeben eines Bestimmungsergebnisses des Pflegemodus an die Mundpflegevorrichtung (100).

15. Programm zum Veranlassen des Computers, das Verfahren zur Bestimmung eines Mundpflegemodus nach Anspruch 14 auszuführen.

## Revendications

1. Dispositif de détermination de mode de soin bucco-dentaire, comprenant :
un détecteur de quantité d'adhésion de plaque dentaire (156) qui détecte, à partir d'une image d'une cavité buccale, une quantité de plaque dentaire qui est une quantité d'adhésion de plaque dentaire (99a) à l'intérieur de la cavité buccale ;
un détecteur de mauvais état (157) qui détecte, à partir de l'image, un niveau de mauvais état qui est un niveau d'un mauvais état d'une gencive à l'intérieur de la cavité buccale ;
un déterminateur de mode de soin (153b) qui détermine, en fonction de la quantité de plaque dentaire détectée et du niveau de mauvais état détecté, un mode de soin qui définit une commande d'entraînement d'un dispositif de soin bucco-dentaire (100) destiné à être utilisé pour soigner la cavité buccale ; et
un dispositif de sortie de résultat (153c) qui délivre un résultat de détermination du mode de soin au dispositif de soin bucco-dentaire (100).

2. Dispositif de détermination de mode de soin bucco-dentaire selon la revendication 1, dans lequel
l'image est une image capturée par détection d'une lumière générée du fait que la cavité buccale est irradiée avec une lumière d'irradiation d'une longueur d'onde prédéterminée à laquelle la plaque dentaire est sensible, et la plaque dentaire à l'intérieur de la cavité buccale répondant à la lumière d'irradiation.

3. Dispositif de détermination de mode de soin bucco-dentaire selon la revendication 1, dans lequel
l'image comprend deux images ou plus qui comprennent une première image montrant une portion de la cavité buccale et une seconde image montrant une autre portion de la cavité buccale,
le détecteur de quantité d'adhésion de plaque dentaire détecte la quantité de plaque dentaire pour chacune de la portion et de l'autre portion de la cavité buccale,
le détecteur de mauvais état détecte le niveau de mauvais état pour chacune de la portion et de l'autre portion de la cavité buccale,
le déterminateur de mode de soin détermine le mode de soin pour chacune de la portion et de l'autre portion de la cavité buccale, et
le dispositif de sortie de résultat
délivre un résultat de détermination du mode de soin pour la portion de la cavité buccale en association avec la portion de la cavité buccale, et
délivre un résultat de détermination du mode de soin pour l'autre portion de la cavité buccale en association avec l'autre portion de la cavité buccale.

4. Dispositif de détermination de mode de soin bucco-dentaire selon la revendication 3, dans lequel
le dispositif de sortie de résultat délivre le résultat de détermination du mode de soin pour la portion de la cavité buccale en association avec une position à l'intérieur de la cavité buccale correspondant à la portion de la cavité buccale, et délivre le résultat de détermination du mode de soin pour l'autre portion de la cavité buccale en association avec une position à l'intérieur de la cavité buccale correspondant à l'autre portion de la cavité buccale.

5. Dispositif de détermination de mode de soin bucco-dentaire selon la revendication 1, dans lequel
le déterminateur de mode de soin détermine le mode de soin en sélectionnant un candidat parmi une pluralité de candidats, et
chacun de la pluralité de candidats pour le mode de soin comprend au moins deux types de paramètres de commande dont chacun définit une commande d'entraînement différente du dispositif de soin bucco-dentaire, les au moins deux types de paramètres de commande étant définis par candidat dans la pluralité de candidats.

6. Dispositif de détermination de mode de soin bucco-dentaire selon la revendication 5, dans lequel
au moins un type de paramètre de commande parmi les au moins deux types de paramètres de commande est un paramètre pour ajuster une performance de nettoyage des dents du dispositif de soin bucco-dentaire, et au moins un autre type de paramètre de commande parmi les au moins deux types de paramètres de commande est un paramètre pour compléter la performance de nettoyage des dents du dispositif de soin bucco-dentaire.

7. Dispositif de détermination de mode de soin bucco-dentaire selon la revendication 1, dans lequel
le dispositif de soin bucco-dentaire est un dispositif de nettoyage bucco-dentaire de type à écoulement de liquide qui éjecte un liquide de nettoyage vers une dent,
le mode de soin comprend un ou plusieurs types de paramètres de commande dont chacun définit une commande d'entraînement différente du dispositif de soin bucco-dentaire,
l'au moins un type de paramètre de commande est au moins l'un parmi un débit, une pression d'éjection, une durée d'éjection ou une fréquence d'éjection du liquide de nettoyage, et
le déterminateur de mode de soin détermine le mode de soin pour amener, en ajustant l'au moins un type de paramètre de commande inclus dans le mode de soin, une charge appliquée à la gencive lors de l'éjection du liquide de nettoyage à être plus petite à mesure que le niveau de mauvais état se détériore.

8. Dispositif de détermination de mode de soin bucco-dentaire selon la revendication 1, dans lequel
le dispositif de soin bucco-dentaire est un dispositif de brosse à dents électrique qui entraîne une brosse prévue sur le dispositif de soin bucco-dentaire,
le mode de soin comprend un ou plusieurs types de paramètres de commande dont chacun définit une commande d'entraînement différente du dispositif de soin bucco-dentaire,
l'au moins un type de paramètre de commande est au moins l'un parmi un couple d'entraînement, une course d'entraînement, une vitesse d'entraînement ou une durée d'entraînement lors de l'entraînement de la brosse, et
le déterminateur de mode de soin détermine le mode de soin pour amener, en ajustant l'au moins un type de paramètre de commande inclus dans le mode de soin, une charge appliquée à la gencive lors de l'entraînement de la brosse à être plus petite à mesure que le niveau de mauvais état se détériore.

9. Dispositif de détermination de mode de soin bucco-dentaire selon la revendication 8, dans lequel le dispositif de soin bucco-dentaire comprend :
un détecteur de pression de brossage qui détecte une pression de brossage appliquée à la brosse ; et
un notificateur d'information de brossage qui notifie à un utilisateur une information de retour concernant la pression de brossage,
le notificateur d'information de brossage définit un ou plusieurs seuils pour la pression de brossage, et lorsque la pression de brossage détectée dépasse un seuil parmi le ou les seuils, le notificateur d'information de brossage fournit à l'utilisateur une notification suggérant de réduire la pression de brossage,
le mode de soin comprend en outre un paramètre concernant le ou les seuils, et
le déterminateur de mode de soin détermine le mode de soin pour amener, en ajustant le paramètre concernant le ou les seuils inclus dans le mode de soin, le ou les seuils à être plus petits à mesure que le niveau de mauvais état se détériore.

10. Dispositif de détermination de mode de soin bucco-dentaire selon la revendication 1, dans lequel
le dispositif de sortie de résultat transmet en outre le résultat de détermination du mode de soin à un dispositif terminal connecté au dispositif de détermination de mode de soin bucco-dentaire, et
le dispositif terminal comprend au moins l'un parmi un afficheur qui affiche le résultat de détermination du mode de soin reçu du dispositif de sortie de résultat ou un notificateur qui notifie par le son le résultat de détermination du mode de soin reçu du dispositif de sortie de résultat.

11. Dispositif de détermination de mode de soin bucco-dentaire selon l'une quelconque des revendications 1 à 10, dans lequel
le niveau de mauvais état est une information d'échelle indiquant, sous forme de valeur numérique, un état d'au moins l'un parmi un gonflement ou un saignement de la gencive.

12. Dispositif de détermination de mode de soin bucco-dentaire selon l'une quelconque des revendications 1 à 10, dans lequel
le mode de soin est déterminé selon une table d'état de santé bucco-dentaire dans laquelle le résultat de détermination du mode de soin est associé à une combinaison d'une information d'échelle indiquant la quantité de plaque dentaire sous forme de valeur numérique et d'une information d'échelle concernant le niveau de mauvais état indiquant, sous forme de valeur numérique, un état d'au moins l'un parmi un gonflement ou un saignement de la gencive.

13. Dispositif de détermination de mode de soin bucco-dentaire selon la revendication 12, dans lequel
lorsque la tendance du changement dans les résultats de détection précédents du niveau de mauvais état montre une détérioration du niveau de mauvais état, le déterminateur de mode de soin met à jour la table d'état de santé bucco-dentaire pour diminuer une performance de soin dans un résultat de détermination.

14. Procédé de détermination de mode de soin bucco-dentaire exécuté par un ordinateur, le procédé de détermination de mode de soin bucco-dentaire comprenant :
la détection, à partir d'une image d'une cavité buccale, d'une quantité de plaque dentaire qui est une quantité d'adhésion de plaque dentaire (99a) à l'intérieur de la cavité buccale ;
la détection, à partir de l'image, d'un niveau de mauvais état qui est un niveau d'un mauvais état d'une gencive (98) à l'intérieur de la cavité buccale ;
la détermination, en fonction de la quantité de plaque dentaire détectée et du niveau de mauvais état détecté, d'un mode de soin qui définit une commande d'entraînement d'un dispositif de soin bucco-dentaire destiné à être utilisé pour soigner la cavité buccale ; et
la délivrance d'un résultat de détermination du mode de soin au dispositif de soin bucco-dentaire (100).

15. Programme pour amener l'ordinateur à exécuter le procédé de détermination de mode de soin bucco-dentaire selon la revendication 14.
